(19) Europäisches Patentamt
European Patent Office
Office européen des brevets

(11) EP 3 988 553 A1

(12) **EUROPEAN PATENT APPLICATION**
published in accordance with Art. 153(4) EPC

(43) Date of publication:
**27.04.2022 Bulletin 2022/17**

(21) Application number: **20827468.8**

(22) Date of filing: **19.06.2020**

(51) International Patent Classification (IPC):
***C07D 498/04*** *(2006.01)* ***A61K 31/517*** *(2006.01)*
***A61P 35/00*** *(2006.01)* ***C07D 403/12*** *(2006.01)*
***C07D 405/14*** *(2006.01)*

(52) Cooperative Patent Classification (CPC):
**A61K 31/517; A61P 35/00; C07D 403/12; C07D 405/14; C07D 498/04**

(86) International application number:
**PCT/CN2020/097158**

(87) International publication number:
**WO 2020/253836 (24.12.2020 Gazette 2020/52)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **19.06.2019 CN 201910533240**

(71) Applicants:
- **Shenzhen Jinrui Foundation Biotech Co., Ltd Guangdong 518118 (CN)**
- **Medshine Discovery Inc. Nanjing, Jiangsu 210032 (CN)**

(72) Inventors:
- **CHEN, Kevin X Shanghai 200131 (CN)**
- **ZHANG, Li Shanghai 200131 (CN)**
- **ZHOU, Kai Shanghai 200131 (CN)**
- **YU, Yanxin Shanghai 200131 (CN)**
- **HU, Boyu Shanghai 200131 (CN)**
- **ZHANG, Huiyu Shanghai 200131 (CN)**

(74) Representative: **De Clercq & Partners Edgard Gevaertdreef 10a 9830 Sint-Martens-Latem (BE)**

# (54) CRYSTAL FORM AND SALT OF QUINAZOLINE COMPOUND AND PREPARATION METHOD THEREFOR

(57) The present invention relates to a crystal form and a salt of a compound serving as a Pan-HER tyrosine kinase inhibitor and a preparation method therefor, and also use thereof in the preparation of a medicament for treating solid tumors.

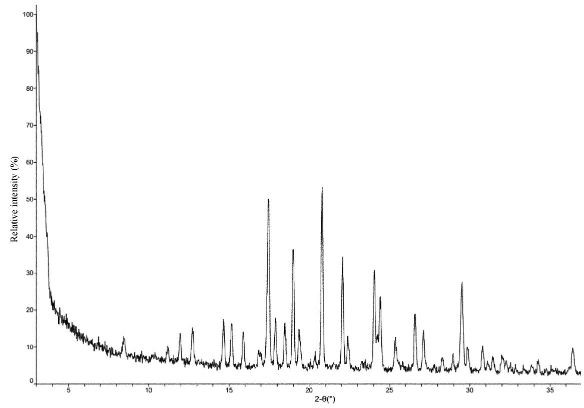


FIG. 1

EP 3 988 553 A1

## Description

This application claims the priority of:
CN201910533240.0, filed on June 19, 2019.

## FIELD OF THE INVENTION

The present disclosure relates to a crystal form and a salt of a compound as a Pan-HER tyrosine kinase inhibitor and a preparation method thereof, and use thereof in the manufacture of a medicament for treating solid tumors.

## BACKGROUND OF THE INVENTION

Human epidermal growth factor receptor (HER, EGFR) is a member of the protein tyrosine kinase family. It is widely distributed on the cell membrane of various human tissues, and can regulate the proliferation, growth, metastasis and apoptosis of cells. Its structure is composed of three parts: extracellular ligand binding region, transmembrane region and intracellular tyrosine kinase region. According to the structural differences of receptors, HER can be divided into four isoforms, i.e., HER1 (EGFR, ErbB-1), HER2 (ErbB-2), HER3 (ErbB-3) and HER4 (ErbB-4). Research disclosed that there is over expression or abnormal activation of HER in various tumor cells such as breast cancer, non-small cell lung cancer, gastric cancer, pancreatic cancer, ovarian cancer, colorectal cancer, head and neck squamous cell carcinoma, malignant glioma and prostate cancer. In addition, research shows that the over expression or abnormal activation of HER is closely associated with the degree of tumor differentiation, the degree of malignancy and prognosis (Baselga. J., Oncologist 2002, 7, 2-8). Therefore, the inhibition of HER has become a hot topic in the research of anti-tumor drugs.

At present, targeted HER inhibitors on the market include Gefitinib, Erlotinib and Lapatinib, etc. However, these marketed drugs have a low effective response rate, are susceptiple to drug resistance, and have some toxic and side effects. Therefore, there is an urgent need to develop other anti-tumor drugs that have excellent anti-tumor effects, can overcome drug resistance, and are well tolerated.

Irreversible inhibitors of Pan-HER tyrosine kinase simultaneously inhibit HER1, HER2 and HER4. Research reveals that this irreversible inhibition of HER family receptors can not only enhance the activity of the drug, but also reduce the occurrence of drug resistance. At the same time, it has a significant inhibitory effect on some tumor cell lines with drug resistance, such as the H1975 cell line that is resistant to Erlotinib. At present, the marketed irreversible inhibitors of Pan-HER tyrosine kinase are only Afatinib and Neratinib. Many inhibitors are in clinical research, such as Poziotinib, Dacomitinib and Canertinib, and there are still unmet market demands.

Therefore, it is necessary to further develop irreversible inhibitors of Pan-HER tyrosine kinase for the treatment of cancer.

Poziotinib (control compound 1) is a Pan-HER inhibitor developed by WO2008150118, with the following structure:

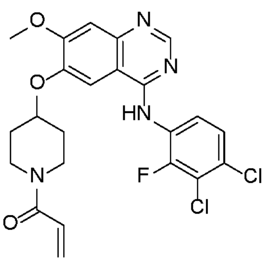

The patent application WO2015043515 discloses control compound 2, which has inhibitory effects on EGFR and HER2, and has the following structure:

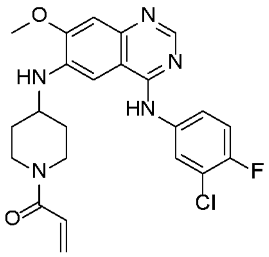

Control compound 2

## SUMMARY OF THE INVENTION

The present disclosure provides a crystal form A of a compound of formula (I), which is characterized in that it has an X-ray powder diffraction pattern having characteristic diffraction peaks at 2θ angles of: 3.3±0.2°, 17.4±0.2°, and 20.8±0.2°.

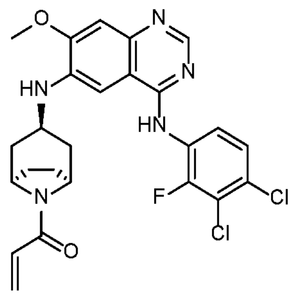

(I)

In some embodiments of the present disclosure, the X-ray powder diffraction pattern of the above-mentioned crystal form A has characteristic diffraction peaks at 2θ angles of: 3.3±0.2°, 14.6±0.2°, 15.2±0.2°, 17.4±0.2°, 19.0±0.2°, 20.8±0.2°, 22.1±0.2°, 24.1±0.2°, and 29.5±0.2°.

In some embodiments of the present disclosure, the X-ray powder diffraction pattern of the above-mentioned crystal form A has characteristic diffraction peaks at 2θ angles of: 3.3±0.2°, 12.0±0.2°, 12.7±0.2°, 14.6±0.2°, 15.2±0.2°, 15.9±0.2°, 17.4±0.2°, 19.0±0.2°, 20.8±0.2°, 22.1±0.2°, 24.1±0.2°, and 29.5±0.2°.

In some embodiments of the present disclosure, the X-ray powder diffraction pattern of the above-mentioned crystal form A has characteristic diffraction peaks at 2θ angles of: 3.3°, 3.7°, 6.9°, 11.2°, 12.0°, 12.7°, 14.6°, 15.2°, 15.9°, 16.9°, 17.4°, 17.9°, 18.5°, 19.0°, 19.4°, 20.1°, 20.4°, 20.8°, 22.1°, 22.4°, 23.5°, 24.1°, 24.4°, 25.4°, 25.8°, 26.6°, 27.1°, 28.3°, 29.0°, and 29.5°.

In some embodiments of the present disclosure, the XRPD pattern of the above-mentioned crystal form A is as shown in FIG. 1.

In some embodiments of the present disclosure, the analysis data of the XRPD pattern of the above-mentioned crystal form A is as shown in Table 1:

**Table 1 Analysis data of the XRPD pattern of the crystal form A of the compound of formula (I)**

| No. | 2θ Angle (°) | D-spacing (Å) | Relative intensity (%) | No. | 2θ Angle (°) | D-spacing (Å) | Relative intensity (%) |
|---|---|---|---|---|---|---|---|
| 1 | 3.285 | 26.8697 | 100 | 16 | 20.086 | 4.417 | 4.1 |
| 2 | 3.68 | 23.9869 | 45.2 | 17 | 20.373 | 4.3556 | 8.7 |
| 3 | 6.876 | 12.8454 | 5.7 | 18 | 20.81 | 4.2651 | 93.1 |
| 4 | 11.19 | 7.9004 | 7.3 | 19 | 22.073 | 4.0237 | 57.3 |
| 5 | 11.957 | 7.3957 | 15.1 | 20 | 22.392 | 3.9672 | 16.8 |
| 6 | 12.725 | 6.951 | 18.7 | 21 | 23.484 | 3.7851 | 3.8 |

(continued)

| No. | 2θ Angle (°) | D-spacing (Å) | Relative intensity (%) | No. | 2θ Angle (°) | D-spacing (Å) | Relative intensity (%) |
|---|---|---|---|---|---|---|---|
| 7 | 14.659 | 6.0377 | 24 | 22 | 24.063 | 3.6952 | 51.2 |
| 8 | 15.168 | 5.8363 | 22.1 | 23 | 24.422 | 3.6418 | 36.9 |
| 9 | 15.881 | 5.576 | 17.9 | 24 | 25.363 | 3.5087 | 16.8 |
| 10 | 16.864 | 5.253 | 7.6 | 25 | 25.824 | 3.4472 | 4.4 |
| 11 | 17.458 | 5.0756 | 85.8 | 26 | 26.607 | 3.3474 | 28.7 |
| 12 | 17.893 | 4.9533 | 24.2 | 27 | 27.122 | 3.2851 | 20.4 |
| 13 | 18.483 | 4.7964 | 21.9 | 28 | 28.269 | 3.1543 | 6.3 |
| 14 | 18.996 | 4.668 | 60.3 | 29 | 28.969 | 3.0797 | 8.1 |
| 15 | 19.351 | 4.5831 | 19.4 | 30 | 29.527 | 3.0228 | 45.6 |

The present disclosure provides a compound of formula (II).

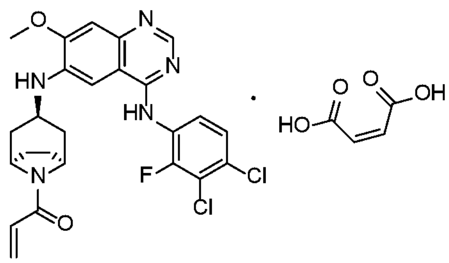

(II)

The present disclosure provides a crystal form B of the compound of formula (II), which is characterized in that it has an X-ray powder diffraction pattern having characteristic diffraction peaks at 2θ angles of: 8.0±0.2°, 11.4±0.2°, and 18.7±0.2°.

The present disclosure provides a crystal form B of the compound of formula (II), which is characterized in that it has an X-ray powder diffraction pattern having characteristic diffraction peaks at 2θ angles of: 12.7±0.2°, 18.7±0.2°, and 24.8±0.2°.

In some embodiments of the present disclosure, the X-ray powder diffraction pattern of the above-mentioned crystal form B has characteristic diffraction peaks at 2θ angles of: 8.0±0.2°, 11.4±0.2°, 12.7±0.2°, 15.0±0.2°, 16.1±0.2°, 18.7±0.2°, 20.0±0.2°, and 20.7±0.2°.

In some embodiments of the present disclosure, the X-ray powder diffraction pattern of the above-mentioned crystal form B has characteristic diffraction peaks at 2θ angles of: 11.4±0.2°, 12.7±0.2°, 16.1±0.2°, 18.7±0.2°, 20.0±0.2°, 22.4±0.2°, 23.1±0.2°, and 24.8±0.2°.

In some embodiments of the present disclosure, the X-ray powder diffraction pattern of the above-mentioned crystal form B has characteristic diffraction peaks at 2θ angles of: 8.0±0.2°, 11.4±0.2°, 12.7±0.2°, 15.0±0.2°, 16.1±0.2°, 18.7±0.2°, 20.0±0.2°, 20.7±0.2°, 22.4±0.2°, 23.1±0.2°, and 24.8±0.2°.

In some embodiments of the present disclosure, the X-ray powder diffraction pattern of the above-mentioned crystal form B has characteristic diffraction peaks at 2θ angles of: 6.825°, 8.041°, 11.412°, 12.72°, 13.454°, 15.007°, 16.067°, 16.817°, 17.402°, 18.009°, 18.691°, 20.012°, 20.748°, 21.265°, 22.03°, 22.383°, 22.779°, 23.075°, 23.648°, 24.475°, 24.77°, 25.593°, 25.914°, 26.248°, 27.076°, 27.527°, 28.557°, and 28.987°.

In some embodiments of the present disclosure, the XRPD pattern of the above-mentioned crystal form B is as shown in FIG. 2.

In some embodiments of the present disclosure, the analysis data of the XRPD pattern of the above-mentioned crystal form B is as shown in Table 2:

**Table 2 Analysis data of the XRPD pattern of the crystal form B of the compound of formula (II)**

| No. | 2θ Angle (°) | D-spacing (Å) | Relative intensity (%) | No. | 2θ Angle (°) | D-spacing (Å) | Relative intensity (%) |
|---|---|---|---|---|---|---|---|
| 1 | 6.825 | 12.9411 | 6.9 | 15 | 22.03 | 4.0315 | 15.8 |
| 2 | 8.041 | 10.9860 | 32.2 | 16 | 22.383 | 3.9687 | 52.2 |
| 3 | 11.412 | 7.7473 | 35.2 | 17 | 22.779 | 3.9006 | 49.9 |
| 4 | 12.72 | 6.9538 | 70.7 | 18 | 23.075 | 3.8512 | 69.3 |
| 5 | 13.454 | 6.5757 | 5.7 | 19 | 23.648 | 3.7592 | 22.1 |
| 6 | 15.007 | 5.8986 | 25.7 | 20 | 24.475 | 3.634 | 29.6 |
| 7 | 16.067 | 5.5117 | 41.8 | 21 | 24.77 | 3.5914 | 75.2 |
| 8 | 16.817 | 5.2675 | 10.7 | 22 | 25.593 | 3.4778 | 26.9 |
| 9 | 17.402 | 5.0917 | 12.5 | 23 | 25.914 | 3.4354 | 29 |
| 10 | 18.009 | 4.9216 | 8.4 | 24 | 26.248 | 3.3924 | 23 |
| 11 | 18.691 | 4.7434 | 100 | 25 | 27.076 | 3.2905 | 46.9 |
| 12 | 20.012 | 4.4333 | 40.3 | 26 | 27.527 | 3.2376 | 25.4 |
| 13 | 20.748 | 4.2776 | 29.6 | 27 | 28.557 | 3.1232 | 31 |
| 14 | 21.265 | 4.1748 | 3.3 | 28 | 28.987 | 3.0778 | 22.1 |

In some embodiments of the present disclosure, the above-mentioned crystal form B has a differential scanning calorimetry curve having an onset of an endothermic peak at 207.4±3.0°C.

In some embodiments of the present disclosure, the DSC curve of the above-mentioned crystal form B is as shown in FIG. 3.

The present disclosure provides a compound of formula (III).

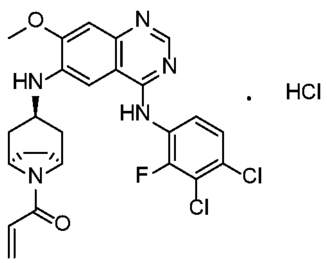

(III)

The present disclosure provides a crystal form C of the compound of formula (III), which is characterized in that it has an X-ray powder diffraction pattern having characteristic diffraction peaks at 2θ angles of: 3.3±0.2°, 7.6±0.2°, and 8.5±0.2°.

The present disclosure provides a crystal form C of the compound of formula (III), which is characterized in that it has an X-ray powder diffraction pattern having characteristic diffraction peaks at 2θ angles of: 3.3±0.2°, 13.8±0.2°, and 17.7±0.2°.

In some embodiments of the present disclosure, the X-ray powder diffraction pattern of the above-mentioned crystal form C has characteristic diffraction peaks at 2θ angles of: 3.3±0.2°, 7.6±0.2°, 8.5±0.2°, 11.3±0.2°, 13.8±0.2°, 15.2±0.2°, 17.7±0.2°, and 18.3±0.2°.

In some embodiments of the present disclosure, the X-ray powder diffraction pattern of the above-mentioned crystal form C has characteristic diffraction peaks at 2θ angles of: 3.3±0.2°, 7.6±0.2°, 11.3±0.2°, 13.8±0.2°, 15.2±0.2°, 17.7±0.2°, 23.7±0.2°, and 24.9±0.2°.

In some embodiments of the present disclosure, the X-ray powder diffraction pattern of the above-mentioned crystal form C has characteristic diffraction peaks at 2θ angles of: 3.3±0.2°, 7.6±0.2°, 8.5±0.2°, 11.3±0.2°, 13.8±0.2°, 15.2±0.2°, 17.7±0.2°, 18.3±0.2°, 23.7±0.2°, 24.1±0.2°, 24.9±0.2°, and 26.6±0.2°.

In some embodiments of the present disclosure, the X-ray powder diffraction pattern of the above-mentioned crystal form C has characteristic diffraction peaks at 2θ angles of: 3.3±0.2°, 7.6±0.2°, 8.5±0.2°, 11.3±0.2°, 13.8±0.2°, 15.2±0.2°, 17.7±0.2°, 18.3±0.2°, 22.6±0.2°, 23.7±0.2°, 24.9±0.2°, and 26.6±0.2°.

In some embodiments of the present disclosure, the X-ray powder diffraction pattern of the above-mentioned crystal form C has characteristic diffraction peaks at 2θ angles of: 3.315°, 3.621°, 7.651°, 8.528°, 10.898°, 11.3°, 13.081°, 13.472°, 13.767°, 15.247°, 15.93°, 16.47°, 17.103°, 17.733°, 18.322°, 18.759°, 19.173°, 19.369°, 20.123°, 20.532°, 21.146°, 21.914°, 22.629°, 22.841°, 23.709°, 24.127°, 24.938°, 25.581°, 26.221°, 26.588°, 27.005°, 27.658°, 28.109°, 28.508°, 29°, and 29.289°.

In some embodiments of the present disclosure, the XRPD pattern of the above-mentioned crystal form C is as shown in FIG. 4.

In some embodiments of the present disclosure, the analysis data of the XRPD pattern of the above-mentioned crystal form C is as shown in Table 3:

**Table 3 Analysis data of the XRPD pattern of the crystal form C of the compound of formula (III)**

| No. | 2θ Angle (°) | D-spacing (Å) | Relative intensity (%) | No. | 2θ Angle (°) | D-spacing (Å) | Relative intensity (%) |
|---|---|---|---|---|---|---|---|
| 1 | 3.315 | 26.632 | 100 | 19 | 20.123 | 4.4091 | 9.3 |
| 2 | 3.621 | 24.3835 | 52.6 | 20 | 20.532 | 4.3222 | 8 |
| 3 | 7.651 | 11.5447 | 32.1 | 21 | 21.146 | 4.198 | 5.6 |
| 4 | 8.528 | 10.3603 | 13.2 | 22 | 21.914 | 4.0526 | 4.6 |
| 5 | 10.898 | 8.1115 | 12.3 | 23 | 22.629 | 3.926 | 13.3 |
| 6 | 11.3 | 7.8241 | 30.5 | 24 | 22.841 | 3.8902 | 11.3 |
| 7 | 13.081 | 6.7626 | 14.3 | 25 | 23.709 | 3.7497 | 29 |
| 8 | 13.472 | 6.5669 | 28.3 | 26 | 24.127 | 3.6857 | 25.6 |
| 9 | 13.767 | 6.4268 | 38.9 | 27 | 24.938 | 3.5675 | 20.2 |
| 10 | 15.247 | 5.8062 | 17.5 | 28 | 25.581 | 3.4793 | 8.4 |
| 11 | 15.93 | 5.5589 | 9.5 | 29 | 26.221 | 3.3959 | 4.7 |
| 12 | 16.47 | 5.3779 | 4.9 | 30 | 26.588 | 3.3498 | 21.7 |
| 13 | 17.103 | 5.1801 | 9.9 | 31 | 27.005 | 3.2991 | 14.4 |
| 14 | 17.733 | 4.9976 | 52.9 | 32 | 27.658 | 3.2225 | 17 |
| 15 | 18.322 | 4.8381 | 13.9 | 33 | 28.109 | 3.1719 | 6.1 |
| 16 | 18.759 | 4.7264 | 4.6 | 34 | 28.508 | 3.1284 | 4.8 |
| 17 | 19.173 | 4.6252 | 7.6 | 35 | 29 | 3.0765 | 12.4 |
| 18 | 19.369 | 4.5789 | 7.2 | 36 | 29.289 | 3.0467 | 5.2 |

The present disclosure provides a crystal form D of the compound of formula (III), which is characterized in that it has an X-ray powder diffraction pattern having characteristic diffraction peaks at 2θ angles of: 3.5±0.2°, 9.5±0.2°, and 10.5±0.2°.

In some embodiments of the present disclosure, the X-ray powder diffraction pattern of the above-mentioned crystal form D has characteristic diffraction peaks at 2θ angles of: 3.5±0.2°, 9.5±0.2°, 10.5±0.2°, 14.0±0.2°, 16.3±0.2°, 18.7±0.2°, 19.0±0.2°, and 23.4±0.2°.

In some embodiments of the present disclosure, the X-ray powder diffraction pattern of the above-mentioned crystal form D has characteristic diffraction peaks at 2θ angles of: 3.5±0.2°, 9.5±0.2°, 10.5±0.2°, 14.0±0.2°, 16.3±0.2°, 18.7±0.2°, 23.4±0.2°, and 25.6±0.2°.

In some embodiments of the present disclosure, the X-ray powder diffraction pattern of the above-mentioned crystal form D has characteristic diffraction peaks at 2θ angles of: 3.502°, 9.505°, 10.509°, 12.166°, 12.446°, 14.008°, 15.385°, 15.684°, 16.275°, 17.495°, 18.681°, 19.016°, 19.404°, 20.356°, 20.954°, 21.802°, 22.489°, 23.019°, 23.457°, 24.245°, 24.977°, 25.583°, 26.253°, 26.688°, 27.359°, 28.425°, 28.762°, 29.821°, 31.581°, 32.411°, 32.907°, 33.155°, 34.579°, and 36.315°.

In some embodiments of the present disclosure, the XRPD pattern of the above-mentioned crystal form D is as shown in FIG. 5.

In some embodiments of the present disclosure, the analysis data of the XRPD pattern of the above-mentioned crystal form D is as shown in Table 4:

**Table 4 Analysis data of the XRPD pattern of the crystal form D of the compound of formula (III)**

| No. | 2θ Angle (°) | D-spacing (Å) | Relative intensity (%) | No. | 2θ Angle (°) | D-spacing (Å) | Relative intensity (%) |
|---|---|---|---|---|---|---|---|
| 1 | 3.502 | 25.2067 | 100 | 18 | 23.019 | 3.8604 | 7.5 |
| 2 | 9.505 | 9.2968 | 11.2 | 19 | 23.457 | 3.7893 | 10.1 |
| 3 | 10.509 | 8.4112 | 19.4 | 20 | 24.245 | 3.6679 | 6.2 |
| 4 | 12.166 | 7.2686 | 2.9 | 21 | 24.977 | 3.5621 | 4.9 |
| 5 | 12.446 | 7.1063 | 4.3 | 22 | 25.583 | 3.479 | 8.1 |
| 6 | 14.008 | 6.3172 | 15.5 | 23 | 26.253 | 3.3918 | 3 |
| 7 | 15.385 | 5.7544 | 5.8 | 24 | 26.688 | 3.3374 | 3.3 |
| 8 | 15.684 | 5.6456 | 2.6 | 25 | 27.359 | 3.2572 | 2.2 |
| 9 | 16.275 | 5.4418 | 12.1 | 26 | 28.425 | 3.1374 | 5.2 |
| 10 | 17.495 | 5.065 | 8.1 | 27 | 28.762 | 3.1013 | 1.8 |
| 11 | 18.681 | 4.7459 | 17.3 | 28 | 29.821 | 2.9936 | 2.3 |
| 12 | 19.016 | 4.6631 | 13.4 | 29 | 31.581 | 2.8307 | 6.4 |
| 13 | 19.404 | 4.5707 | 3.2 | 30 | 32.411 | 2.76 | 1.9 |
| 14 | 20.356 | 4.3591 | 3.5 | 31 | 32.907 | 2.7196 | 2.7 |
| 15 | 20.954 | 4.2361 | 7.3 | 32 | 33.155 | 2.6998 | 3.2 |
| 16 | 21.802 | 4.0731 | 2.6 | 33 | 34.579 | 2.5918 | 1.6 |
| 17 | 22.489 | 3.9503 | 3.4 | 34 | 36.315 | 2.4718 | 2.4 |

The present disclosure provides a compound of formula (IV).

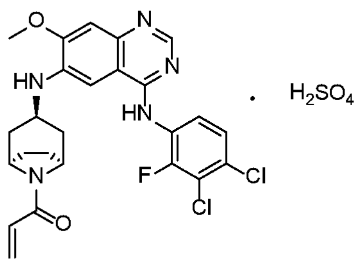

(IV)

The present disclosure provides a crystal form E of the compound of formula (IV), which is characterized in that it has an X-ray powder diffraction pattern having characteristic diffraction peaks at 2θ angles of: 3.3±0.2°, 12.8±0.2°, and 13.2±0.2°.

The present disclosure provides a crystal form E of the compound of formula (IV), which is characterized in that it has an X-ray powder diffraction pattern having characteristic diffraction peaks at 2θ angles of: 3.3±0.2°, 16.2±0.2°, and 23.2±0.2°.

In some embodiments of the present disclosure, the X-ray powder diffraction pattern of the above-mentioned crystal form E has characteristic diffraction peaks at 2θ angles of: 3.3±0.2°, 12.8±0.2°, 13.2±0.2°, 14.9±0.2°, 16.2±0.2°, 18.7±0.2°, 23.2±0.2°, and 24.8±0.2°.

In some embodiments of the present disclosure, the X-ray powder diffraction pattern of the above-mentioned

crystal form E has characteristic diffraction peaks at 2θ angles of: 3.3±0.2°, 8.1±0.2°, 13.2±0.2°, 14.9±0.2°, 16.2±0.2°, 18.7±0.2°, 23.2±0.2°, and 24.8±0.2°.

In some embodiments of the present disclosure, the X-ray powder diffraction pattern of the above-mentioned crystal form E has characteristic diffraction peaks at 2θ angles of: 3.305°, 3.601°, 8.088°, 11.28°, 12.762°, 13.177°, 14.206°, 14.875°, 16.256°, 17.264°, 18.661°, 19.993°, 21.397°, 21.934°, 23.196°, 24.755°, 25.209°, 27.381°, 28.76°, 30.802°, 32.96°, and 33.325°.

In some embodiments of the present disclosure, the XRPD pattern of the above-mentioned crystal form E is as shown in FIG. 6.

In some embodiments of the present disclosure, the analysis data of the XRPD pattern of the above-mentioned crystal form E is as shown in Table 5:

**Table 5 Analysis data of the XRPD pattern of the crystal form E of the compound of formula (IV)**

| No. | 2θ Angle (°) | D-spacing (Å) | Relative intensity (%) | No. | 2θ Angle (°) | D-spacing (Å) | Relative intensity (%) |
|---|---|---|---|---|---|---|---|
| 1 | 3.305 | 26.711 | 100 | 12 | 19.993 | 4.4374 | 4.8 |
| 2 | 3.601 | 24.519 | 51.5 | 13 | 21.397 | 4.1493 | 4.8 |
| 3 | 8.088 | 10.9228 | 9.1 | 14 | 21.934 | 4.049 | 4.4 |
| 4 | 11.28 | 7.838 | 6.3 | 15 | 23.196 | 3.8314 | 35.3 |
| 5 | 12.762 | 6.9309 | 12.6 | 16 | 24.755 | 3.5935 | 17.2 |
| 6 | 13.177 | 6.7135 | 11 | 17 | 25.209 | 3.5299 | 9.6 |
| 7 | 14.206 | 6.2295 | 5.6 | 18 | 27.381 | 3.2546 | 5.1 |
| 8 | 14.875 | 5.9507 | 24.9 | 19 | 28.76 | 3.1015 | 9.8 |
| 9 | 16.256 | 5.4481 | 55.4 | 20 | 30.802 | 2.9004 | 3.1 |
| 10 | 17.264 | 5.1321 | 4.7 | 21 | 32.96 | 2.7153 | 3.3 |
| 11 | 18.661 | 4.7511 | 20.3 | 22 | 33.325 | 2.6864 | 3 |

The present disclosure provides a crystal form F of the compound of formula (IV), which is characterized in that it has an X-ray powder diffraction pattern having characteristic diffraction peaks at 2θ angles of: 3.6±0.2°, 7.5±0.2°, and 16.6±0.2°.

The present disclosure provides a crystal form F of the compound of formula (IV), which is characterized in that it has an X-ray powder diffraction pattern having characteristic diffraction peaks at 2θ angles of: 3.6±0.2°, 12.2±0.2°, and 16.6±0.2°.

In some embodiments of the present disclosure, the X-ray powder diffraction pattern of the above-mentioned crystal form F has characteristic diffraction peaks at 2θ angles of: 3.6±0.2°, 7.5±0.2°, 12.2±0.2°, 15.1±0.2°, 16.6±0.2°, 23.6±0.2°, and 24.6±0.2°.

In some embodiments of the present disclosure, the X-ray powder diffraction pattern of the above-mentioned crystal form F has characteristic diffraction peaks at 2θ angles of: 3.62°, 6.981°, 7.501°, 10.896°, 11.18°, 12.198°, 13.734°, 14.789°, 15.111°, 16.611°, 18.146°, 19.586°, 20.104°, 20.375°, 20.924°, 21.084°, 21.537°, 22.111°, 22.614°, 23.638°, 24.614°, and 25.077°.

In some embodiments of the present disclosure, the XRPD pattern of the above-mentioned crystal form F is as shown in FIG. 7.

In some embodiments of the present disclosure, the analysis data of the XRPD pattern of the above-mentioned crystal form F is as shown in Table 6:

**Table 6 Analysis data of the XRPD pattern of the crystal form F of the compound of formula (IV)**

| No. | 2θ Angle (°) | D-spacing (Å) | Relative intensity (%) | No. | 2θ Angle (°) | D-spacing (Å) | Relative intensity (%) |
|---|---|---|---|---|---|---|---|
| 1 | 3.62 | 24.387 | 100 | 12 | 19.586 | 4.5288 | 3.9 |
| 2 | 6.981 | 12.6512 | 4.4 | 13 | 20.104 | 4.4131 | 4.1 |
| 3 | 7.501 | 11.7764 | 7.1 | 14 | 20.375 | 4.355 | 3.6 |

(continued)

| No. | 2θ Angle (°) | D-spacing (Å) | Relative intensity (%) | No. | 2θ Angle (°) | D-spacing (Å) | Relative intensity (%) |
|---|---|---|---|---|---|---|---|
| 4 | 10.896 | 8.113 | 4.7 | 15 | 20.924 | 4.242 | 3.6 |
| 5 | 11.18 | 7.9074 | 6.6 | 16 | 21.084 | 4.2101 | 4.3 |
| 6 | 12.198 | 7.2502 | 13.6 | 17 | 21.537 | 4.1226 | 3.6 |
| 7 | 13.734 | 6.4423 | 3.9 | 18 | 22.111 | 4.0169 | 3.3 |
| 8 | 14.789 | 5.985 | 3.8 | 19 | 22.614 | 3.9286 | 4.2 |
| 9 | 15.111 | 5.8581 | 13.2 | 20 | 23.638 | 3.7608 | 11.4 |
| 10 | 16.611 | 5.3323 | 22.9 | 21 | 24.614 | 3.6138 | 11.2 |
| 11 | 18.146 | 4.8847 | 4.3 | 22 | 25.077 | 3.5481 | 3.8 |

The present disclosure provides a method for preparing the crystal form A of the compound of formula (I), comprising:

(1) dissolving the compound of formula (I) in a solvent; and
(2) heating with stirring, cooling, filtering, and drying to give the crystal form A of the compound of formula (I).

In some embodiments of the present disclosure, in the above-mentioned method for preparing the crystal form A, the solvent is selected from methanol, ethanol and ethyl acetate.

The present disclosure provides a method for preparing the crystal form B of the compound of formula (II), comprising:

(1) dissolving the compound of formula (I) in a solvent; and
(2) adding maleic acid, and then stirring, filtering, and drying to give the crystal form B of the compound of formula (II).

In some embodiments of the present disclosure, in the above-mentioned method for preparing the crystal form B, the solvent is selected from tetrahydrofuran.

In some embodiments of the present disclosure, in the above-mentioned method for preparing the crystal form B, the stirring temperature ranges from 35°C to 45°C.

In some embodiments of the present disclosure, in the above-mentioned method for preparing the crystal form B, the stirring time ranges from 24 hours to 48 hours.

In some embodiments of the present disclosure, in the above-mentioned method for preparing the crystal form B, the weight-volume ratio of the compound to the solvent ranges from 1g: 10 to 50mL.

The present disclosure provides another method for preparing the crystal form B of the compound of formula (II), comprising:

(1) dissolving the compound of formula (I) in a solvent; and
(2) heating, adding maleic acid, stirring, precipitating solid, cooling, filtering, and drying to give the crystal form B of the compound of formula (II).

In some embodiments of the present disclosure, in the above-mentioned method for preparing the crystal form B, the solvent is selected from ethanol and ethyl acetate.

In some embodiments of the present disclosure, in the above-mentioned method for preparing the crystal form B, the stirring temperature ranges from 65°C to 75°C.

In some embodiments of the present disclosure, in the above-mentioned method for preparing the crystal form B, the stirring time ranges from 16 hours to 24 hours.

In some embodiments of the present disclosure, in the above-mentioned method for preparing the crystal form B, the weight-volume ratio of the compound to the solvent ranges from 1g: 6 to 10mL.

The present disclosure provides a method for preparing the crystal form C of the compound of formula (III), comprising:

(1) adding a compound of formula (I) and hydrochloride thereof to a solvent to give a suspension; and

(2) heating, stirring, centrifuging, and drying to give the crystal form C of the compound of formula (III).

In some embodiments of the present disclosure, in the above-mentioned method for preparing the crystal form C, the solvent is selected from acetonitrile.
In some embodiments of the present disclosure, in the above-mentioned method for preparing the crystal form C, the stirring temperature ranges from 35°C to 45°C.
In some embodiments of the present disclosure, in the above-mentioned method for preparing the crystal form C, the stirring time ranges from 24 hours to 48 hours.
In some embodiments of the present disclosure, in the above-mentioned method for preparing the crystal form C, the weight-volume ratio of the compound to the solvent ranges from 1g: 7 to 10mL.
The present disclosure provides a method for preparing the crystal form D of the compound of formula (III), comprising:

(1) adding a compound of formula (I) and hydrochloride thereof to a solvent to give a suspension; and
(2) heating, stirring, centrifuging, and drying to give the crystal form D of the compound of formula (III).

In some embodiments of the present disclosure, in the above-mentioned method for preparing the crystal form D, the solvent is selected from ethyl acetate, acetonitrile-water (1:1), and methanol-water (1:1).
In some embodiments of the present disclosure, in the above-mentioned method for preparing the crystal form D, the stirring temperature ranges from 35°C to 45°C.
In some embodiments of the present disclosure, in the above-mentioned method for preparing the crystal form D, the stirring time ranges from 24 hours to 48 hours.
In some embodiments of the present disclosure, in the above-mentioned method for preparing the crystal form D, the weight-volume ratio of the compound to the solvent ranges from 1g: 7 to 10mL.
The present disclosure also provides use of the above-mentioned crystal form in the manufacture of a medicament for treating a disease related to a Pan-HER tyrosine kinase inhibitor.
The present disclosure also provides use of the above-mentioned compound or crystal form in the manufacture of a medicament for treating a disease related to a Pan-HER tyrosine kinase inhibitor.
In some embodiments of the present disclosure, the above-mentioned use is characterized in that the medicament related to Pan-HER tyrosine kinase inhibitor is used for a tumor.

**Definition and description**

Unless otherwise specified, the following terms and phrases used herein are intended to have the following meanings. A specific term or phrase should not be considered indefinite or unclear in the absence of a particular definition, but should be understood in the conventional sense. When a trade name appears herein, it is intended to refer to the corresponding commodity or active ingredient thereof.
The intermediate compounds of the present disclosure can be prepared by a variety of synthetic methods well known to those skilled in the art, including specific embodiments listed below, embodiments formed by combining the specific embodiments listed below with other chemical synthetic methods, and equivalent alternative methods well known to those skilled in the art. The alternative embodiments include, but are not limited to, the examples of the present disclosure.
The chemical reactions in the specific embodiments disclosed herein are completed in a suitable solvent, which must be suitable for the chemical changes of the present disclosure and the reagents and materials required. In order to give the compound of the present disclosure, it is sometimes necessary for those skilled in the art to modify or select synthetic steps or reaction schemes based on the existing embodiments.
The present disclosure will be described in detail below through examples, which are not intended to limit the present disclosure in any way.
All solvents used in the present disclosure are commercially available and can be used without further purification.
Solvents used in the present disclosure are commercially available. The following abbreviations are used in the present disclosure: DCM represents dichloromethane; DMF represents *N*,*N*-dimethylformamide; DMSO represents dimethyl sulfoxide; EtOH represents ethanol; MeOH represents methanol; TFA represents trifluoroacetic acid; ATP represents adenosine triphosphate; HEPES represents 4-hydroxyethylpiperazine ethanesulfonic acid; and $MgCl_2$ represents magnesium dichloride.

**Technical effect**

The crystal forms of the compound disclosed herein have good stability, and are easy to be made into a medicament. The compounds disclosed herein have significant inhibitory activity on HER1, HER2 and HER4, and have significant inhibitory activity on the proliferation of NCI-N87, OE21, and OE19 cells. The compounds disclosed herein have excellent pharmacokinetic properties. The compounds disclosed herein have significant inhibitory activity on tumor growth, and have no significant effect on the body weight of mice and have better safety at an effective dose.

**1.1 X-ray powder diffractometer, XRPD**

Instrument model: Bruker D8 advance X-ray diffractometer
Test method: About 10 to 20 mg of sample was used for XRPD detection.
Detailed XRPD parameters were as follows:

Light tube: Cu, Cu: K-Alpha ($\lambda$=1.54179Å).
Tube voltage: 40 kV, tube current: 40 mA.
Scattering slit:0.60 mm
Detector slit: 10.50 mm
Anti-scatter slit: 7.10 mm
Scan range (2$\theta$ angle): 3 or 4 to 40 deg
Scan rate: 10 deg/min
Rotation speed of sample disk: 15 rpm/0 rpm

**1.2 Differential Scanning Calorimeter, DSC**

Instrument model: DSC Q2000 Differential Scanning Calorimeter
Test method: The sample (0.5 to 1 mg) was weighed and placed in a DSC aluminum pot for testing. The sample was heated from 30°C to 300°C at a heating rate of 10°C/min under the condition of 25mL/min $N_2$.

**1.3 High performance liquid chromatography analysis method**

Sample concentration: 0.5 mg/mL
Chromatographic conditions of the HPLC method for solid stability assay were shown in Table 7 below:

**Table 7**

| | |
|---|---|
| Column | XBridge C18 (4.6mm*150mm, 3.5μm) |
| Wavelength | 240nm |
| Column temperature | 40°C |
| Flow rate | 1.0mL/min |
| Injection volume | 10.0μL |
| Mobile phase | Mobile phase A: 20mM aqueous ammonium acetate solution<br>Mobile phase B: acetonitrile |

(continued)

| | | | |
|---|---|---|---|
| Column | XBridge C18 (4.6mm*150mm, 3.5μm) | | |
| Gradient program | Time/minute | A% | B% |
| | 0.01 | 70 | 30 |
| | 40.00 | 40 | 60 |
| | 45.00 | 10 | 90 |
| | 45.10 | 70 | 30 |
| | 50.00 | 70 | 30 |
| | 50.01 | stop | |
| Data collection time | 50min | | |
| Diluent | Acetonitrile:water = 50 : 50(v/v) | | |
| ProbeWash | Acetonitrile:water = 50 : 50(v/v) | | |

## BRIEF DESCRIPTION OF THE DRAWINGS

FIG. 1 is an XRPD pattern from Cu-Ka radiation of the crystal form A of the compound of formula (I).
FIG. 2 is an XRPD pattern from Cu-Ka radiation of the crystal form B of the compound of formula (II).
FIG. 3 is a DSC curve of the crystal form B of the compound of formula (II).
FIG. 4 is an XRPD pattern from Cu-Ka radiation of the crystal form C of the compound of formula (III).
FIG. 5 is an XRPD pattern from Cu-Ka radiation of the crystal form D of the compound of formula (III).
FIG. 6 is an XRPD pattern from Cu-Ka radiation of the crystal form E of the compound of formula (IV).
FIG. 7 is an XRPD pattern from Cu-Ka radiation of the crystal form F of the compound of formula (IV).

## DETAILED DESCRIPTION OF THE INVENTION

In order to better understand the content of the present disclosure, the present disclosure is further illustrated below in conjunction with specific examples, but the specific examples are not intended to limit the content of the present disclosure.

### Example 1: Preparation of the compound of formula (I)

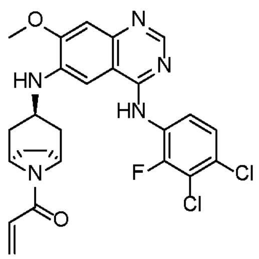

Route for synthesis:

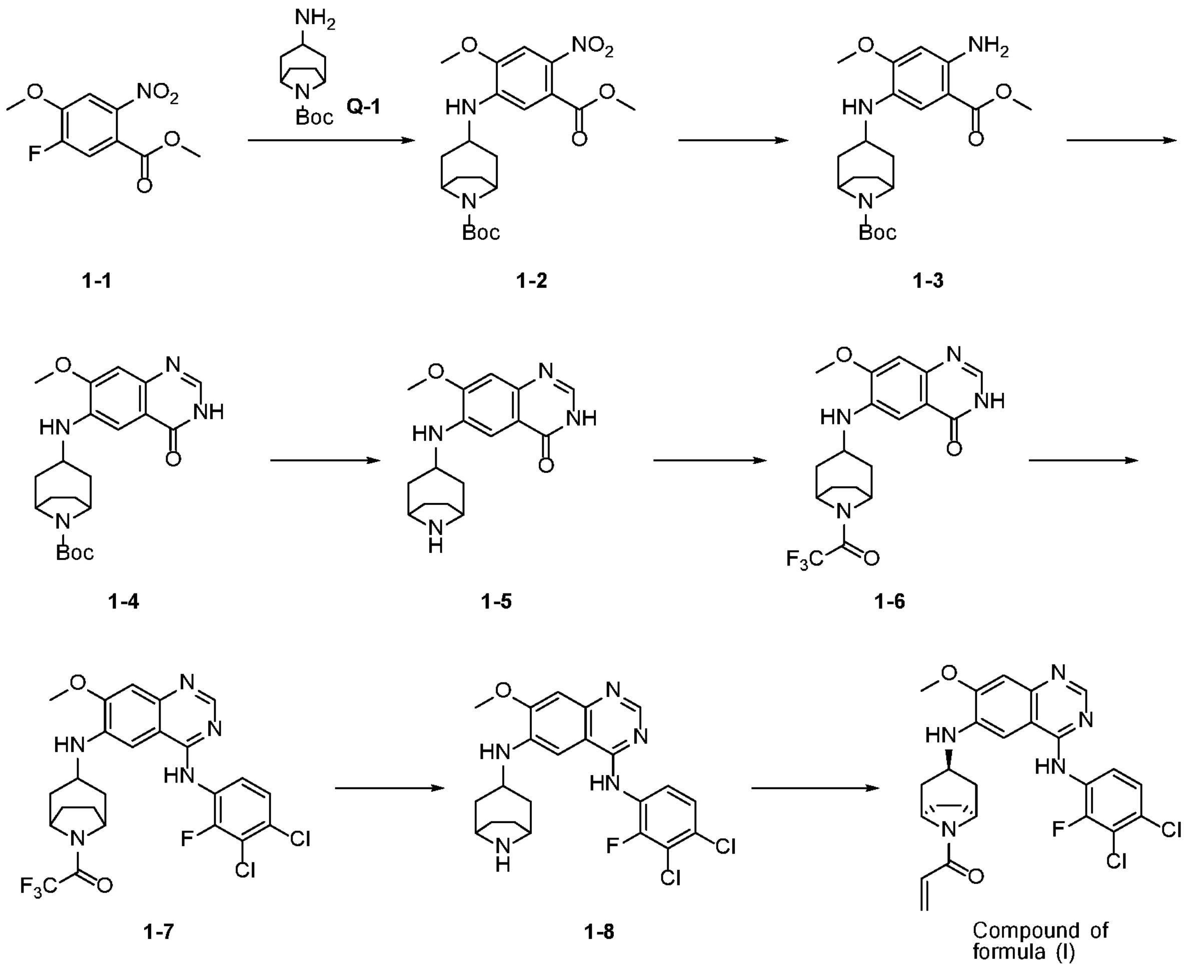

Q-1

1-1 1-2 1-3

1-4 1-5 1-6

1-7 1-8 Compound of formula (I)

The first step

Compound **1-1** (900.0 mg, 3.93 mmol) was dissolved in N, N-dimethylformamide (20 mL), and to the mixture were added potassium carbonate (1.09 g, 7.85 mmol) and compound **Q-1** (1.60 g, 7.07 mmol). The reaction solution was stirred at 75 °C for 16 hours. The reaction solution was cooled to 20 °C, diluted with water (30 mL), and extracted with ethyl acetate (30 mL × 2). The organic phases were combined, washed sequentially with water (40 mL × 2) and saturated brine (40 mL × 2), dried over anhydrous sodium sulfate, and filtered. The filtrate was concentrated under reduced pressure, and the residue was isolated by silica gel column chromatography (ethyl acetate : petroleum ether = 1 : 3) to give compound **1-2.** $^1$H NMR (400 MHz, $CDCl_3$) δ 7.42 (s, 1H), 6.45 (s, 1H), 4.75 (d, *J* = 8.4 Hz, 1H), 4.10-4.40 (m, 2H), 3.87 (s, 3H), 3.86 (s, 3H), 3.75-3.80 (m, 1H), 1.90-2.10 (m, 4H), 1.50-1.70 (m, 4H), 1.41 (s, 9H). LC-MS: *m/z* = 458.1 $[M+Na]^+$.

The second step

Compound **1-2** (1.50 g, 3.44 mmol) was dissolved in methanol (30 mL), and to the mixture was added wet palladium/carbon (10%, 50.0 mg). The reaction solution was stirred at 20 °C under hydrogen atmosphere for 0.5 hours. The reaction solution was filtered, and the filtrate was concentrated under reduced pressure and dried to give compound **1-3**.$^1$H NMR (400 MHz, $CDCl_3$) δ 7.09 (s, 1H), 6.10 (s, 1H), 5.36 (brs, 2H), 4.15-4.40 (m, 2H), 3.85 (s, 3H), 3.82 (s, 3H), 3.65-3.80 (m, 1H), 1.95-2.15 (m, 4H), 1.90-1.85 (m, 2H), 1.47 (s, 9H), 1.30-1.45 (m, 2H). LC-MS: *m/z* = 406.2 $[M+H]^+$.

The third step

Compound **1-3** (1.30 g, 3.21 mmol) and ammonium acetate (2.47 g, 32.06 mmol) were added to trimethyl orthoformate (31.29 g, 294.89 mmol). The reaction solution was stirred at 70 °C for 10 hours. The reaction solution was cooled to 20 °C, and concentrated under reduced pressure. The residue was slurried with water (10 mL) and filtered. The solid was dried to give compound **1-4**. $^1$H NMR (400 MHz, DMSO-$d_6$) δ 7.83 (s, 1H), 7.08 (s, 1H), 7.00 (s, 1H), 5.17

(d, $J$ = 8.4 Hz, 1H), 4.10-4.20 (m, 2H), 3.95-4.00(m, 1H), 3.92 (s, 3H), 1.90-2.00 (m, 4H), 1.75-1.90 (m, 2H), 1.50-1.60 (m, 2H), 1.43 (s, 9H). LC-MS: $m/z$ = 401.1 $[M+H]^+$.

The fourth step

Compound **1-4** (1.20 g, 3.00 mmol) was added to a solution of hydrogen chloride in dioxane (4 N, 10 mL). The reaction solution was stirred at 0 °C for 0.4 hours. The reaction solution was concentrated under reduced pressure to give the hydrochloride salt of compound **1-5.** LC-MS: $m/z$ = 301.0 $[M+H]^+$.

The fifth step

The hydrochloride salt of **1-5** (1.10 g, 3.27 mmol) and triethylamine (4.6 mL, 32.66 mmol) were dissolved in dichloromethane (30 mL) at 0 °C, and to the mixture was added trifluoroacetic anhydride (1.37 g, 6.53 mmol) dropwise. The reaction solution was stirred at 20 °C for 1 hour. The reaction solution was concentrated under reduced pressure, and the residue was slurried with water (40 mL) and filtered. The solid was dried to give compound 1-6. $^1$H NMR (400 MHz, DMSO-$d_6$) $\delta$ 11.90 (s, 1H), 7.80-7.90 (m, 1H), 7.13 (m, 1H), 7.00 (m, 1H), 5.28 (d, $J$ = 9.2 Hz, 1H), 4.60-4.70 (m, 1H), 4.40-4.50 (m, 1H), 4.00-4.10 (m, 1H), 3.91 (s, 3H), 1.90-2.20 (m, 6H), 1.55-1.75 (m, 2H). LC-MS: $m/z$ = 397.0 $[M+H]^+$.

The sixth step

Compound **1-6** (500.0 mg, 1.26 mmol) was dissolved in phosphorus oxychloride (24.1 mL, 260.22 mmol), and to the mixture was added N, N-dimethylformamide (92.2 mg, 1.26 mmol). The reaction solution was stirred at 100 °C for 5 hours. The reaction solution was concentrated under reduced pressure, and to the residue was added a solution of 3, 4-dichloro-2-fluoroaniline (340.6 mg, 1.89 mmol) in isopropanol (5 mL). The reaction solution was stirred at 90 °C for 1 hour, and then cooled to 20 °C. The reaction solution was quenched by adding saturated sodium bicarbonate aqueous solution (10 mL), and extracted with ethyl acetate (10 mL $\times$ 2). The organic phases were combined, dried over anhydrous sodium sulfate, and filtered. The filtrate was concentrated under reduced pressure, and the residue was isolated by silica gel column chromatography (ethyl acetate : petroleum ether = 2 : 1) to give compound **1-7**. $^1$H NMR (400 MHz, $CDCl_3$) $\delta$ 8.50-8.65 (m, 2H), 7.40-7.50 (m, 1H), 7.15-7.25 (m, 2H), 6.59 (s, 1H), 4.75-4.85 (m, 1H), 4.55-4.65 (m, 2H), 4.05-4.10 (m, 1H), 3.98 (s, 3H), 2.10-2.40 (m, 4H), 1.90-2.00 (m, 2H), 1.60-1.75 (m, 2H). LC-MS: $m/z$ = 557.9 $[M+H]^+$.

The seventh step

Compound **1-7** (305.0 mg, 547.2 $\mu$mol) was dissolved in methanol (15 mL), and to the mixture was added potassium carbonate (378.2 mg, 2.74 mmol). The reaction solution was stirred at 50 °C for 14 hours. The reaction solution was concentrated under reduced pressure, and the residue was isolated using a silica gel preparation plate (dichloromethane : methanol = 10 : 1) to give compound **1-8**.$^1$H NMR (400 MHz, DMSO-$d_6$) $\delta$ 9.45 (s, 1H), 8.28 (s, 1H), 7.67 (t, $J$ = 8.0 Hz, 1H), 7.58 (d, $J$ = 10.0 Hz, 1H), 7.20 (s, 1H), 7.14 (s, 1H), 5.17 (d, $J$ = 8.4 Hz, 1H), 4.05-4.10 (m, 2H), 3.98-4.05 (m, 1H), 3.93 (s, 3H), 2.15-2.30 (m, 4H), 1.95-2.10 (m, 2H), 1.70-1.80 (m, 2H). LC-MS: $m/z$ = 462.0 $[M+H]^+$.

The eighth step

Compound **1-8** (220.0 mg, 475.8 $\mu$mol) was dissolved in a mixed solvent of tetrahydrofuran (6 mL) and water (6 mL) at 0 °C. To the mixture was added sodium bicarbonate (119.9 mg, 1.43 mmol), and added slowly acryloyl chloride (38.8 mg, 428.3 $\mu$mol) dropwise. The reaction solution was further stirred at 0 °C for 0.5 hours. The reaction solution was quenched by adding MeOH (1 mL) and concentrated under reduced pressure. The residue was isolated by preparative thin-layer chromatography (dichloromethane : methanol = 10 : 1) to give the **compound of formula (I)**. $^1$H NMR (400 MHz, DMSO-$d_6$) $\delta$ 9.35 (s, 1H), 8.24 (s, 1H), 7.66 (t, $J$ = 8.0 Hz, 1H), 7.57 (d, $J$ = 8.8 Hz, 1H), 7.14 (s, 1H), 7.06 (s, 1H), 6.72 (dd, $J_1$ = 16.8 Hz, $J_2$ = 10.4 Hz, 1H), 6.18 (dd, $J_1$ = 16.8 Hz, $J_2$=1.6 Hz, 1H), 5.68 (dd, $J_1$= 10.4 Hz, $J_2$= 1.6 Hz, 1H), 5.35 (d, $J$ = 9.2 Hz, 1H), 4.50-4.60 (m, 2H), 4.05-4.15 (m, 1H), 3.92 (s, 3H), 1.85-2.20 (m, 6H), 1.60-1.70 (m, 1H), 1.50-1.60 (m, 1H). LC-MS: $m/z$ = 538.0 $[M+Na]^+$.

**Example 2:**

**Preparation of the crystal form A of the compound of formula (I)**

Compound of formula (I) (50 g) was added to a mixed solution of methanol and ethyl acetate (v:v=50 mL:250

mL), and the resulting suspension was heated and stirred at 80°C-90°C. The mixture was slowly cooled down, and then filtered. The solid was dried under reduced pressure to give the crystal form A of the compound of formula (I).

Preparation of the crystal form B of the compound of formula (II)

Compound of formula (I) (40.9 g) was dissolved in ethanol (320 mL), and the mixture was heated to 70°C. A solution of maleic acid (9.4 g) in ethanol (80 mL) was added with stirring. The reaction solution became clear, and then a solid precipitated out. The mixture was stirred at 70°C for 0.5 hours. Ethyl acetate (400 mL) was added, and the mixture was stirred at 70°C for another 16 hours. The mixture was cooled to room temperature, and filtered. The filter cake was collected, and dried under reduced pressure to give the crystal form B of the compound of formula (II).

About 500 mg of the compound of formula (I) was weighed, added to a 40 mL glass bottle, and dissolved by adding 25 mL of tetrahydrofuran. A magnet bar was added, and then the above sample was placed on a magnetic heating stirrer (40°C, 890 rpm) and reacted. A mixture of maleic acid and solvent (maleic acid:acetone = 200mg:1500 μL) was then slowly added, and the mixture was continued to react on the magnetic heating stirrer (40°C, 890rpm). The sample as a suspension (after being magnetically stirred at 40°C and 890rpm for 24 hours) was centrifuged at 3000 rpm for 5 minutes, and then the resulting solid sample was dried in a vacuum drying oven at 30°C to give the crystal form B of the compound of formula (II).

An appropriate amount of the compound of formula (I), about 50 mg of maleate, was weighed and added to a 2.0 mL glass vial, and an appropriate amount of solvent (see Table 8 below) was added to obtain a suspension. A magnet bar was added, and the above sample was placed on a magnetic heating stirrer. After shaking at 40°C for 2 days, the above sample was centrifuged, and the resulting solid sample was dried in a vacuum drying oven at 35°C overnight to give the crystal form B of the compound of formula (II).

**Table 8 Preparation of the crystal form B of the compound of formula (II)**

| Batch No. | No. | Amount of the compound of formula (I) added (mg) | Solvent | Amount of the solvent added (μL) | State | Crystal form |
|---|---|---|---|---|---|---|
| Maleate | 01 | 50.087 | Ethanol | 600 | Suspens ion | B |
| | 02 | 50.252 | Water | 400 | Suspens ion | B |
| | 03 | 50.243 | Ethyl acetate | 400 | Suspens ion | B |
| | 04 | 50.254 | Acetonitrile: water (1:1) | 400 | Suspens ion | B |
| | 05 | 50.234 | Methanol: water (1:1) | 400 | Suspens ion | B |

**Preparation of the crystal form C and crystal form D of the compound of formula (III)**

An appropriate amount of the compound of formula (I), about 20-30 mg of hydrochloride, was weighed and added to a 2.0 mL glass vial, and an appropriate amount of solvent (see Table 9 below) was added to obtain a suspension. A magnet bar was added, and then the above sample was placed on a magnetic heating stirrer. After shaking at 40°C for 2 days, the above sample was centrifuged, and the resulting solid sample was dried in a vacuum drying oven at 35°C overnight to give the crystal form C or crystal form D of the compound of formula (III).

**Table 9 Preparation of the crystal form C and crystal form D of the compound of formula (III)**

| Batch No. | No. | Amount of the compound of formula (I) added (mg) | Solvent | Amount of the solvent added (μL) | State | Crystal form |
|---|---|---|---|---|---|---|
| Hydrochloride | 01 | 30.8 | Acetonitrile | 200 | Suspension | C |
| | 02 | 50.110 | Ethyl acetate | 400 | Suspension | D |
| | 03 | 50.189 | Acetonitrile: water (1:1) | 400 | Suspension | D |
| | 04 | 50.003 | Methanol :water (1:1) | 400 | Suspension | D |

**Preparation of the crystal form E of the compound of formula (IV)**

25 mg of the compound of formula (I), about 20-30 mg of sulfate, was weighed and added to a 2.0 mL glass vial, and 200μL of methanol was added to obtain a suspension. A magnet bar was added, and the above sample was placed on a magnetic heating stirrer. After shaking at 40°C for 2 days, the above sample was centrifuged, and the resulting solid sample was dried in a vacuum drying oven at 35°C overnight to give the crystal form E of the compound of formula (IV).

**Preparation of the crystal form F of the compound of formula (IV)**

50.284 mg of the compound of formula (I), about 50 mg of sulfate, was weighed and added to a 2.0 mL glass vial, and 200μL of ethanol was added to obtain a suspension. A magnet bar was added, and the above sample was placed on a magnetic heating stirrer. After shaking at 40°C for 2 days, the above sample was centrifuged, and the resulting solid sample was dried in a vacuum drying oven at 35°C overnight to give the crystal form F of the compound of formula (IV).

**Example 3: Solid stability assay of the crystal form B of the compound of formula (I)**

According to influencing factor and accelerated assay conditions, about 5 mg of the crystal form B of the compound of formula (I) was accurately weighed and placed in a dry and clean glass bottle to form a thin layer as a formal assay sample in duplicate. The sample was placed under an influencing factor assay condition (60 °C, 92.5% RH) and accelerated conditions (40 °C/75% RH and 60 °C/75% RH). The sample was fully exposed, and covered with aluminum foil paper punched with small holes. In addition, a small amount of sample was placed in a 40 mL glass sample bottle under the same conditions to determine the crystal state. The sample placed under light irradiation condition ($1.2\times10^6$ Lux•hr/near ultraviolet 200w•hr/$m^2$) was fully exposed at room temperature. The assay results were shown in Table 10.

**Table 10 Results of the solid stability assay of the crystal form B of the compound of formula (I)**

| Assay conditions | Time | Crystal form (XRPD) | Total impurity content (%) |
|---|---|---|---|
| Initial | 0 day | B | 1.64 |
| High temperature (60°C, open) | 5 days | B | 1.64 |
| | 10 days | B | 1.64 |
| High humidity (25°C/92.5% relative humidity, open) | 5 days | B | 1.65 |
| | 10 days | B | 1.65 |
| Protected from light | 10 days | B | 1.64 |
| Light irradiation (total illuminance 1.2×10°Lux•hr/near ultraviolet 200w•hr/$m^2$, open) | 10 days | B | 1.66 |

(continued)

| Assay conditions | Time | Crystal form (XRPD) | Total impurity content (%) |
|---|---|---|---|
| 40°C, 75% relative humidity, open | 10 days | B | 1.64 |
| | 1 month | B | 1.62 |
| | 2 months | B | 1.52 |
| | 3 months | B | 1.52 |
| 60°C, 75% relative humidity, open | 10 days | B | 1.63 |
| | 1 month | B | 1.62 |

**Conclusion:** The crystal form B of the compound of formula (I) has good stability.

**Biochemical assay: in vitro evaluation**

**Assay example 1: evaluation of enzyme activity**

The purpose of this assay is to detect the in vitro inhibitory activity of compounds on HER1(ErbB1), HER2(ErbB2), and HER4(ErbB4). The enzymes used in this assay are human ErbB 1, ErbB2 and ErbB4. Eurofins Pharma Discovery Service provided the method for activity detection. The results of inhibitory activity of the assay compounds on HER1, HER2, and HER4 are shown in Table 11.

Steps and method of the assay (96-well plate):
A buffer solution of the assay compounds in a dilution of 5-fold (5μL), polypeptide substrate poly(Glu, Tyr) (4:1) (2.5μL), ErbB (4-20 ng, 2.5μL), $MnCl_2$ (50 mM, 1.25 μL), $dH_2O$ (3.75 μL), and [γ-$^{33}$P]ATP (10 μL) were added, and incubated at 30 °C for 10 minutes. The reaction was terminated by adding 3% phosphoric acid. 10 μL of the sample was taken and transferred to Filtermate A. The filter disc was washed 3 times with 75 mM phosphoric acid and once with methanol. The filter disc was transferred to a sealed plastic bag and the scintillation mixture (4 mL) was added. The intensity of the emitted photon was detected by a scintillation luminescence counter. The CPM (times/min) of the enzyme sample was compared with the CPM of the internal control sample. The level of photon intensity reflected the degree of the tyrosine kinase activity.

**Table 11: results of the in vitro enzyme activity screening assay for the compound of formula (I)**

| Compound | **HER1 $IC_{50}$ (nM)** | **HER2 $IC_{50}$ (nM)** | **HER4 $IC_{50}$ (nM)** |
|---|---|---|---|
| Compound of formula (I) | 8 | 5 | 3 |

Conclusion: the compound of formula (I) has significant inhibitory activity on HER1, HER2 and HER4.

Assay example 2: evaluation of inhibitory activity on cell proliferation

Purpose of the assay: the inhibitory activity of the assay compounds on cell proliferation was detected.

Principle of the assay: the luciferase in a Cell-Titer-Glo reagent produces oxidized luciferin with luciferin, oxygen and ATP as reaction substrates, and releases energy in the form of light. Since the luciferase reaction requires ATP, the total amount of light generated by the reaction is proportional to the total amount of ATP that reflects cell viability.

Materials of the assay:

Cell line: NCI-N87 cell line (ATCC-CRL-5822), BT-474 cell line (ATCC-HTB-20), OE21 (ECACC-96062201)
Cell culture medium: (RPMI 1640 medium (Invitrogen#22400-105; 10% serum Invitrogen #10090148; L-glutamine 1×, Gibco#25030-081; penicillin-streptomycin Hyclone# SV30010) Cell Titer-Glo® cell viability assay kit with luminescence (Promega #G7573)
384-well cell culture plate (Greiner # 781090)
Compound plate (LABCYTE # LP-0200)
CO2 incubator (Thermo#371)
Vi-cell cell counter (Beckman Coulter)

Pipette (Eppendorf)
Pipetting tube (Greiner)
Pipetting gun (Eppendorf)
Multifunctional microplate reader (Envision Reader)
ECHO Liquid-handling workstation (Labcyte-ECHO555)

Steps and method of the assay:

2.1 Day 1:
According to the schematic diagram of the cell seed plate, cells were seeded in a 384- or 96-well plate at a density of 1000 cells per well and 25 μL per well, and 25 μL of PBS was added to the wells near edge of the place where no cells were seeded.
2.2 Day 0:

(1) The stock solution of the compounds has a concentration of 10 mM, and the compounds were diluted with DMSO to an initial concentration of 4 mM. The compounds were added to the plate with stock solution of compounds, 9 μL per well.
(2) The compounds were diluted with an ECHO liquid workstation, where 125 nL of compounds were added to each well of the cell plate, 125 nL of DMSO was added to each of the cell well in columns 2 and 23, and 125 nL of DMSO was added to each PBS well in columns 1 and 24.
(3) The cell plate was supplemented with 25 μL of medium per well, to a final volume of 50 μL per well; the compounds had a concentration of 1 μM, and was serially 3-fold diluted to obtain 10 concentrations, and the left and right wells were used in duplicate; the final concentration of DMSO was 0.25%.

2.3 After addition of the compounds, the cell plate was centrifuged at 1000 rpm for 1 min, and then placed in a 37 °C, 5% $CO_2$ incubator for incubation for 3 days.
2.4 Day 3:
The cell plate was taken out from the incubator and equilibrated at room temperature for 30 minutes. 25 μL of Cell-Titer-Glo reagent was added to each well, shaken for one minute to allow well mixed, and centrifuged at 1000 rpm for 1 minute. After 10 minutes, the plate was read on PerkinElmer Envision, and the fluorescence reading time was set to 0.2 second.

Assay results: the assay results are shown in Table 12.

CTG assay of OE19 cells

OE19 cells with saturation of 80%-90% were digested with trypsin, centrifuged, resuspended and counted. The concentration of cells was adjusted to 90 ml/well. OE19 cells were added to a 96-well cell culture plate to 8000 OE19 cells per well. The cells were cultured in a cell incubator containing 5% $CO_2$ at 37 °C overnight.
The stock solution of the assay compounds was serially 3-fold diluted with DMSO to a total of 10 concentrations. Before the start of the assay, the serially diluted assay compounds were further diluted to 10 × compound solutions (the highest concentration of 100μM containing 1% DMSO) with cell culture medium under sterile conditions. The prepared 10 × compound solutions were added to the cell culture plate with 10 μL per well. In this way, the final concentrations of staurosporine as the standard control and all the assay compounds were obtained starting from 10 μM as the initial concentration in a 3-fold serial dilution to get 10 assay concentrations.
After 72 hours of cell culture, the 96-well cell culture plate was taken out. Cell Titer Glo reagent was added at 50 ml/well, mixed, centrifuged, and incubated at room temperature in the dark for 10 minutes. The cell plate was placed in Envision for reading.
The inhibition rate was calculated according to original data as follows:

$$\text{Inhibition \%} = (\text{ZPE} - \text{sample detection value})/(\text{ZPE}-\text{HPE}) \times 100\%$$

The well containing 10 mM staurosporine on DAY3 (the first day of the assay is the day when compounds were added, and DAY3 is the day when reading is perfomed) was used as the HPE (100% inhibition control) well, and the well containing 0.1% DMSO on DAY3 was used as the ZPE (0% inhibition control) well. Each concentration of assay compounds was assayed in duplicate.

Non-linear regression analysis was performed using the processed data and GraphPad Prism 6 analysis software to obtain a dose-response curve, and the half-inhibitory concentration ($IC_{50}$) of the assay compounds on OE19 cells was calculated. The assay results are shown in Table 2.

**Table 12: results of the screening assay of inhibitory activity of the compound of formula (I) on cell proliferation in vitro**

| Compound | NCI-N87 cell | OE21 cell[c] | OE19 cell[c] |
|---|---|---|---|
| | $IC_{50}$ (nM) | $IC_{50}$ (nM) | $IC_{50}$ (nM) |
| Compound of formula (I) | 0.46 | 0.52 | 1.20 |

Conclusion: the compound of formula (I) has significant inhibitory activity on the proliferation of NCI-N87, OE21 and OE19 cells.

Assay example 3: in vivo pharmacodynamics study in BALB/c nude mouse model with subcutaneous xenograft tumor of human gastric cancer NCI-N87 cells

Purpose of the assay: in vivo efficacy of the assay compound disclosed herein on subcutaneous xenograft tumor of human gastric cancer NCI-N87 cells in a BALB/c nude mouse model was evaluated.
Animals of the assay: female BALB/c nude mice, 6-8 weeks old, weighted 18-22 grams; supplier: Shanghai Ling Chang Biotechnology Co., Ltd.

Method and steps of the assay:

3.1 cell culture

Human gastric cancer NCI-N87 cells were cultured in monolayer in vitro. The culture conditions were RPMI-1640 medium plus 10% fetal bovine serum, 100 U/mL penicillin, 100 U/mL streptomycin and 2 mM glutamine, 37 °C, 5 % $CO_2$. The passaging was performed by the conventional digestion with trypsin-EDTA twice a week. When the cell saturation was 80%-90%, the cells were collected, counted and inoculated.

3.2 Tumor cell inoculation (tumor inoculation)

0.2 mL ($10 \times 10^6$) of NCI-N87 cells (PBS + Matrigel, 1:1) were inoculated subcutaneously on the right back of each nude mouse. When the average tumor volume reached 158 $mm^3$, administration to each group was started.

3.3 Formulation of assay compounds

The assay compounds were formulated into 0.04 mg/mL and 0.08 mg/mL clear solution in the solvents of 10% NMP (N-methylpyrrolidone) + 10% ethylene glycol stearate + 80% water.

3.4 Tumor measurement and assay index

The assay index was to investigate whether tumor growth is inhibited, delayed or cured. Tumor diameter was measured twice a week with a vernier caliper. The calculation formula of tumor volume was: $V = 0.5a \times b^2$, wherein a and b represent the long and short diameters of tumor, respectively.
The antitumor efficacy of the compounds was evaluated by TGI (%) or tumor proliferation rate T/C (%). TGI (%) reflects tumor growth inhibition rate. TGI (%) was calculated as follows: TGI (%) = [1-(average tumor volume at the end of administration for a certain treatment group - average tumor volume at the beginning of administration for this treatment group)/(average tumor volume at the end of treatment for a vehicle control group - average tumor volume at the beginning of administration for this vehicle control group)] × 100%.
Tumor proliferation rate T/C (%): the calculation formula was as follows: T/C(%) = average tumor volume at the end of administration for a certain treatment group /average tumor volume at the end of treatment for a vehicle control group × 100%.

3.5 Statistical analysis

Statistical analysis included mean and standard error of mean (SEM) of tumor volume at each time point in each group (See Table 13 for specific data). The treatment group showed the best therapeutic effect at the end of the assay, i.e., on the 28th day after administration. Therefore, based on this data, statistical analysis was performed to assess the difference between groups. The comparison between groups was analyzed by T-test, and the comparison among three or more groups was analyzed by one-way ANOVA. After testing, F value showed a significant difference, and the test was carried out by Games-Howell method. All data analysis was carried out with SPSS 17.0. $p<0.05$ was considered a significant difference.

3.6 Assay results

3.6.1 Mortality, morbidity and body weight changes

The body weight of assay animals was used as a reference index for indirect determination of drug toxicity. In this model, the body weight of mice in the treatment group had a downward trend, and there was no other morbidity or death.

3.6.2 Evaluation index of antitumor efficacy

**Table 13: evaluation of anti-tumor efficacy of the compound of formula (I) in subcutaneous xenograft tumor model of human gastric cancer NCI-N87 cells (calculated based on the tumor volume on the 28th day after administration)**

| Group | Dose | Tumor volume (mm3)[a] (Day 28) | T/C[b] (%) | TGI[b] (%) | *p value*[c] |
|---|---|---|---|---|---|
| Vehicle group | -- | 626 ± 60 | -- | -- | -- |
| Poziotinib | 0.8mg/kg | 28 ± 8 | 4.47 | 127.78 | *0.002* |
| Poziotinib | 0.4mg/kg | 84 ± 9 | 13.42 | 115.81 | *0.003* |
| Compound of formula (I) | 0.8mg/kg | 15 ± 3 | 2.40 | 130.56 | *0.002* |
| Compound of formula (I) | 0.4mg/kg | 47 ± 13 | 7.51 | 123.72 | *0.002* |

Note: a. Mean + SEM.
b. Tumor growth inhibition was calculated by T/C and TGI (TGI (%) = [1-(T28-T0)/(V28-V0)]×100).
c. The p value was calculated based on tumor volume.

3.7 Conclusion and discussion of assay

In the assay, in vivo efficacy of the compound of formula (I) in subcutaneous xenograft tumor model of human gastric cancer NCI-N87 cells was evaluated. Compared with the vehicle control group, the compound of formula (I) @0.8 mg/kg in the treatment group was comparable to the reference compound Poziotinib@0.8 mg/kg in terms of the drug efficacy, both of which exhibited significant tumor inhibitory effect. In the low-dose group, the compound of formula (I) was comparable to or better than the reference compound Poziotinib in terms of the drug efficacy.

**Assay example 4: in vivo pharmacodynamics study in BALB/c nude mouse model with subcutaneous xenograft tumor of human gastric cancer NCI-N87 cells**

Purpose of the assay: in vivo efficacy of the assay compound disclosed herein on subcutaneous xenograft tumor of human gastric cancer NCI-N87 cells in a BALB/c nude mouse model was evaluated.
Animals of the assay: female BALB/c nude mice, 6-8 weeks old, weighted 18-22 grams; supplier: shanghai sippr bk laboratory animals Ltd.

Method and steps of the assay:

4.1 cell culture

Human gastric cancer NCI-N87 cells were cultured in monolayer in vitro. The culture conditions were RPMI-1640 medium plus 10% fetal bovine serum, 100 U/mL penicillin, 100 μg/mL streptomycin, 37 °C, 5 % $CO_2$. The passaging was performed by conventional digestion with trypsin-EDTA twice a week. When the cell saturation was 80%-90%, the cells were collected, counted and inoculated.

4.2 Tumor cell inoculation (tumor inoculation)

0.2 mL ($10 \times 10^6$) of NCI-N87 cells (PBS + Matrigel, 1:1) were inoculated subcutaneously on the right back of each mouse. When the average tumor volume reached 156 $mm^3$, administration to each group was started.

4.3 Formulation of assay compounds

The assay compounds were formulated into a 0.05 mg/mL clear solution in a solvent of 10% NMP (N-methylpyrrolidone) + 10% ethylene glycol stearate + 80% water.

4.4 Tumor measurement and assay index

The assay index was to investigate whether tumor growth is inhibited, delayed or cured. Tumor diameter was measured twice a week with a vernier caliper. The calculation formula of tumor volume was: $V = 0.5a \times b^2$, wherein a and b represent the long and short diameters of tumor, respectively.

The antitumor efficacy of the compounds was evaluated by TGI (%) or tumor proliferation rate T/C (%). TGI (%) reflects the tumor growth inhibition rate. TGI (%) was calculated as follows: TGI (%) = [1-(average tumor volume at the end of administration for a certain treatment group - average tumor volume at the beginning of administration for this treatment group)/(average tumor volume at the end of treatment for a vehicle control group - average tumor volume at the beginning of administration for this vehicle control group)]×100%.

Tumor proliferation rate T/C (%): the calculation formula was as follows: T/C (%) = average tumor volume at the end of administration for a certain treatment group/average tumor volume at the end of treatment for the vehicle control group × 100%.

4.5 Statistical analysis

Statistical analysis included the mean and standard error of mean (SEM) of tumor volume at each time point in each group (See Table 14 for specific data). The treatment group showed the best therapeutic effect at the end of the assay, i.e., on the twenty-eight day after administration. Therefore, based on this data, statistical analysis was performed to assess the difference between the groups. The comparison between groups was analyzed by T-test, and the comparison among three or more groups was analyzed by one-way ANOVA. After testing, F value showed significant difference, and thus a test was carried out by Games-Howell method. All data analysis was carried out with SPSS 17.0. $p<0.05$ was considered a significant difference.

4.6 Assay results

4.6.1 Mortality, morbidity and body weight changes

The body weight of assay animals was used as a reference index for indirect determination of drug toxicity. In this model, the body weight of mice in the treatment group had a downward trend, and there was no other morbidity or death.

4.6.2 Evaluation index of antitumor efficacy

**Table 14: evaluation of anti-tumor efficacy of the compound of formula (I) in subcutaneous xenograft tumor model of human gastric cancer NCI-N87 cells (calculated based on the tumor volume on the21st day after administration)**

| Group | Dose | Tumor volume ($mm^3$)[a] (Day 21) | T/C[b] (%) | TGI[b] (%) | *p value*[c] |
|---|---|---|---|---|---|
| Vehicle group | -- | 631 ± 107 | -- | -- | -- |
| Compound of formula (I) | 0.5mg/kg | 63 ± 10 | 10.69 | 119.41 | *0.007* |
| Control compound 2 | 0.5mg/kg | 541 ± 88 | 89.35 | 18.83 | *0.796* |

Note: a. Mean + SEM.
b. Tumor growth inhibition was calculated by T/C and TGI (TGI (%) = [1-(T21-T0)/(V21-V0)]×100).
c. The p value was calculated based on tumor volume.

4.7 Conclusion and discussion of assay

In the assay, in vivo efficacy of the compound of formula (I) and control compound 2 in subcutaneous xenograft tumor model of human gastric cancer NCI-N87 cells was evaluated. Compared with the vehicle control group, the compound of formula (I)@0.5 mg/kg in the treatment group exhibited significant tumor inhibitory effect, in which the tumor inhibition rate was T/C = 10.69 %, TGI = 119.41 %, and p = 0.007; at the same dose, the efficacy of control compound 2 was not significant, in which T/C = 89.35 %, and TGI = 18.83 %; the efficacy of the compound of formula (I) was significantly better than that of the control compound 2.

**Assay example 5: in vivo pharmacodynamics study in BALB/c nude mouse model with subcutaneous xenograft tumor of human esophageal carcinoma OE21 cells**

Purpose of the assay: in vivo efficacy of the assay compound on subcutaneous xenograft tumor of human esophageal carcinoma OE21 cells in a BALB/c nude mouse model was evaluated.

Animals of the assay: female BALB/c nude mice, 6-8 weeks old, weighted 17-23 grams; supplier: shanghai sippr bk laboratory animals Ltd.

Method and steps of the assay:

5.1 cell culture

Human esophageal carcinoma OE21 cells were cultured in monolayer in vitro. The culture conditions were RPMI-1640 medium plus 10% fetal bovine serum, 100 U/mL penicillin, 100 U/mL streptomycin and 2 mM glutamine, 37 °C, 5 % $CO_2$. The passaging was performed by conventional digestion with trypsin-EDTA twice a week. When the cell saturation was 80%-90%, the cells were collected, counted and inoculated.

5.2 Tumor cell inoculation (tumor inoculation)

0.1 ml (20.1) OE21 cells (PBS) were inoculated subcutaneously on the right back of each mouse. When the average tumor volume reached 112 $mm^3$, administration to each group was started.

5.3 Formulation of assay compounds

The assay compound was formulated into a 0.15 mg/mL to 0.3 mg/mL clear or suspension solution in the solvents of 10% NMP + 10% ethylene glycol stearate + 80% water.

5.4 Tumor measurement and assay index

The assay index was to investigate whether tumor growth is inhibited, delayed or cured. Tumor diameter was measured twice a week with vernier caliper. The calculation formula of tumor volume was: $V = 0.5a \times b^2$, wherein a and b represent the long and short diameters of tumor, respectively.

The antitumor efficacy of the compounds was evaluated by TGI (%) or relative tumor proliferation rate T/C (%).

TGI (%) reflects the tumor growth inhibition rate. TGI (%) was calculated as follows: TGI (%) = [(1-(average tumor volume at the end of administration for a certain treatment group - average tumor volume at the beginning of administration for this treatment group))/(average tumor volume at the end of treatment for a vehicle control group - average tumor volume at the beginning of administration for this vehicle control group)] × 100%.

Relative tumor proliferation rate T/C (%): the calculation formula was as follows: T/C (%) = average tumor volume at the end of administration for a certain treatment group/average tumor volume at the end of treatment for the vehicle control group × 100%.

5.5 Statistical analysis

Statistical analysis included the mean and standard error of mean (SEM) of tumor volume at each time point in each group. One-way ANOVA was used for comparison among three or more groups. If F value showed significant difference, multiple comparison should be made after ANOVA analysis. SPSS 17.0 was used for all data analysis. $p<0.05$ was considered a significant difference.

5.6 Assay results

5.6.1 Mortality, morbidity and body weight changes

In this model, animals in the administration groups of Poziotinib@2/1.5 mg/kg and the compound of formula (I) @2/1.5 mg/kg all showed varying degrees of body weight loss and dandruff; in the Poziotinib@2/1.5 mg/kg administration group, from the 6th and 7th day after the administration to each group, 1 mouse showed more than 15% of body weight loss and then 3 mice showed more than 15% of body weight loss, and their administration was discontinued; moreover, mice in all groups had dark yellow urine, which suggested toxicity; starting from the 3rd day after administration, the dose of Poziotinib@2 mg/kg was reduced to 1.5 mg/kg, and the dose of the compound of formula (I)@2 mg/kg was reduced to 1.5 mg/kg. After the dose adjustment, the body weight of animals in each group recovered significantly.

5.6.2 Evaluation index of antitumor efficacy

**Table 15: evaluation of anti-tumor efficacy of the compound of formula (I) on OE21 xenograft tumor model (calculated based on tumor volume on day 21 after administration)**

| Group | Dose | Tumor volume (mm3)a (Day 21) | T/C[b] (%) | TGI[b] (%) | *p value*[c] |
|---|---|---|---|---|---|
| Vehicle group | | 948±124 | -- | -- | -- |
| Poziotinib | 2 /1.5mg/kg | 57±7 | 6.05 | 106.65 | *0.008* |
| Compound of formula (I) | 2 /1.5mg/kg | 55±13 | 5.82 | 106.65 | *0.008* |

Note: a. Mean + SEM.
b. Tumor growth inhibition was calculated by T/C and TGI (referring to Assay example 4).
c. The p value was calculated based on the tumor volume on the 21st day after administration using One-Way ANOVA statistical method by comparing with the vehicle group.

5.7 Conclusion and discussion of assay

In this assay, in vivo efficacy of the compound of formula (I) and the reference compound Poziotinib in the OE21 xenograft tumor model was evaluated. On the 21st day after the administration, the tumor volume of tumor-bearing mice in the vehicle control group reached 948 $mm^3$. The assay substance Poziotinib@2/1.5 mg/kg and the compound of formula (I)@2/1.5 mg/kg group had a significant tumor inhibitory effect compared with the vehicle control group. The tumor inhibition rate of Poziotinib@2/1.5 mg/kg group was T/C = 6.05 %, TGI = 106.65 %, and p = 0.008; and the tumor inhibition rate of the compound of formula (I)@2/1.5 mg/kg group was T/C = 5.82 %, TGI = 106.65 %, and p = 0.008. The efficacy of the compound of formula (I) at a low dose of 0.5 mpk was comparable to that of the reference compound Poziotnib and the compound of formula (I) at a high dose of 1.5 mpk.

The compound of formula (I) had significant tumor inhibitory effect, and the efficacy of the compound of formula (I) was comparable to that of the reference compound Poziotinib. However, in the high-dose group of the reference

compound, the body weight loss of mice was significant. Three mice showed more than 15% of body weight loss, and their administration was discontinued. Moreover, mice in the whole group had dark yellow urine, which suggested toxicity. The compound of formula (I) had little effect on the body weight of mice, without discontinuing the administration and without the toxicity suggestion of dark yellow urine, suggesting that its tolerability was significantly better than that of the reference compound. Compared with the reference compound Poziotinib, the compound of formula (I) had lower effective dose, better tolerability, and superior safety window.

**Assay example 6: assay to study pharmacokinetics in mice**

Purpose of the assay: this assay aimed to investigate in vivo plasma pharmacokinetics of the example compound disclosed herein and the reference compound in female BALB/c mice after the single intravenous injection and intragastric administration thereof.

Animals of the assay: female BALB/c nude mice, 7-9 weeks old, weighted 17-23 grams; supplier: shanghai sippr bk laboratory animals Ltd.

Sample collection: at each time point, a 0.03 mL blood sample was collected from the saphenous vein of the assay animal by puncture, and the actual blood collection time was recorded. All blood samples were added into commercial EDTA-K2 anticoagulation tubes with a specification of 1.5 mL (the supplier was Jiangsu KANGJIAN Medical Apparatus Co., Ltd.). Within half an hour after the blood sample was collected, the blood sample was centrifuged at 3000g at 4 °C for 10 minutes, and the supernatant plasma was drawn, quickly placed in dry ice, and stored in a refrigerator at -80 °C for LC-MS/MS analysis.

Data analysis: Plasma concentrations were processed using a non-compartmental model of WinNonlin™Version6.3 (Pharsight, MountainView, CA) pharmacokinetic software, and pharmacokinetic parameters $C_{max}$, $T_{max}$, $T_{1/2}$, and $AUC_{0-last}$ were calculated using a linear log trapezoid method.

**Table 16: comparison of PK results of the compound of formula (I) and the reference compound**

| | $C_{max}$ (nM) | $T_{max}$ (h) | $T_{1/2}$ (h) | $AUC_{0-last}$ (nM.h) |
|---|---|---|---|---|
| Compound of formula (I) | 10600 | 2.0 | 3.2 | 122154 |
| Poziotinib | 17267 | 0.25 | 2.3 | 97870 |
| Control compound 2 | 15300 | 2.0 | 1.5 | 82021 |

$C_{max}$: peak concentration; $T_{max}$: time to reach peak concentration; $T_{1/2}$: the time required to clear half of compound; $AUC_{0-last}$: integrated area of concentration during the time from 0 to the last sampling.

Conclusion: the results of pharmacokinetic studies in mice showed that the compound of formula (I) had a longer half-life than that of the reference compound, and had significantly better oral plasma exposure than that of the reference compound Poziotinib and the control compound 2.

**Assay example 7: evaluation of inhibition of CYP enzyme activity**

The results of the compound disclosed herein and the reference compound Poziotinib in the assay of CYP enzyme activity in human liver microsome were shown in the following table (Table 17).

**Table 17: comparison of results of the compound of formula (I) and the reference compound on the inhibition of CYP enzyme**

| | CYP1A2 | CYP2C9 | CYP2C19 | CYP2D6 | CYP3A4 |
|---|---|---|---|---|---|
| Compound of formula (I) | >50 | 16 | 11 | 6 | >50 |
| Poziotinib | >50 | 8 | 12 | 1 | >50 |

The results of inhibition of CYP activity in human liver microsome by the compound disclosed herein showed that the compound of formula (I) and Poziotinib had no inhibitory activity on CYP1A2 and CYP3A4. The inhibitory activity of the compound of formula (I) on CYP2C19 was comparable to that of the reference compound Poziotinib. The activity on CYP2C9 and CYP2D6 was improved by the compound of formula (I), and the activity of the compound of formula (I) on CYP2C9 and CYP2D6 was 2 times and 6 times better than that of the reference compound, respectively. By comprehensive comparison, the compound of formula (I) had improved activity on CYP, which was superior to the activity of the reference compound on CYP. Therefore, the risk of drug-drug interaction was lower.

## Claims

1. A crystal form A of a compound of formula (I), which is **characterized in that** it has an X-ray powder diffraction pattern having characteristic diffraction peaks at 2θ angles of: 3.3±0.2°, 17.4±0.2°, and 20.8±0.2°

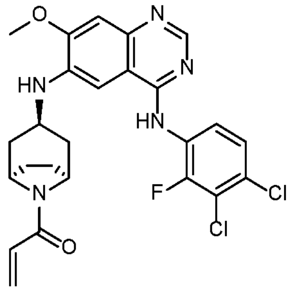

(I)

.

2. The crystal form A according to claim 1, wherein the X-ray powder diffraction pattern has characteristic diffraction peaks at 2θ angles of 3.3±0.2°, 14.6±0.2°, 15.2±0.2°, 17.4±0.2°, 19.0±0.2°, 20.8±0.2°, 22.1±0.2°, 24.1±0.2°, and 29.5±0.2°.

3. The crystal form A according to claim 2, wherein the X-ray powder diffraction pattern has characteristic diffraction peaks at 2θ angles of 3.3±0.2°, 12.0±0.2°, 12.7±0.2°, 14.6±0.2°, 15.2±0.2°, 15.9±0.2°, 17.4±0.2°, 19.0±0.2°, 20.8±0.2°, 22.1±0.2°, 24.1±0.2°, and 29.5±0.2°.

4. The crystal form A according to claim 3, wherein the X-ray powder diffraction pattern has characteristic diffraction peaks at 2θ angles of 3.3°, 3.7°, 6.9°, 11.2°, 12.0°, 12.7°, 14.6°, 15.2°, 15.9°, 16.9°, 17.4°, 17.9°, 18.5°, 19.0°, 19.4°, 20.1°, 20.4°, 20.8°, 22.1°, 22.4°, 23.5°, 24.1°, 24.4°, 25.4°, 25.8°, 26.6°, 27.1°, 28.3°, 29.0°, and 29.5°.

5. The crystal form A according to claim 4, wherein the XRPD pattern is as shown in FIG. 1.

6. A compound of formula (II),

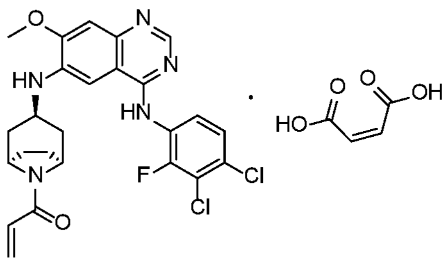

(II)

.

7. A crystal form B of the compound of formula (II), which is **characterized in that** it has an X-ray powder diffraction pattern having characteristic diffraction peaks at 2θ angles of: 8.0±0.2°, 11.4±0.2°, and 18.7±0.2°.

8. The crystal form B according to claim 7, wherein the X-ray powder diffraction pattern has characteristic diffraction peaks at 2θ angles of 8.0±0.2°, 11.4±0.2°, 12.7±0.2°, 15.0±0.2°, 16.1±0.2°, 18.7±0.2°, 20.0±0.2°, and 20.7±0.2°.

9. The crystal form B according to claim 8, wherein the X-ray powder diffraction pattern has characteristic diffraction peaks at 2θ angles of 8.0±0.2°, 11.4±0.2°, 12.7±0.2°, 15.0±0.2°, 16.1±0.2°, 18.7±0.2°, 20.0±0.2°, 20.7±0.2°, 22.4±0.2°, 23.1±0.2°, and 24.8±0.2°.

10. The crystal form B according to claim 9, wherein the XRPD pattern is as shown in FIG. 2.

**11.** The crystal form B according to any one of claims 7 to 10, wherein it has a differential scanning calorimetry curve having an onset of an endothermic peak at 207.4±3.0°C.

**12.** The crystal form B according to claim 11, wherein the DSC curve is as shown in FIG. 3.

**13.** A compound of formula (III),

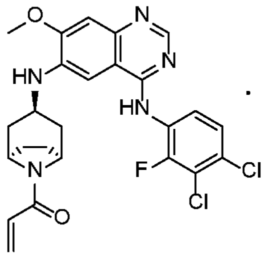

· HCl

(III)

.

**14.** A crystal form C of the compound of formula (III), which is **characterized in that** it has an X-ray powder diffraction pattern having characteristic diffraction peaks at 2θ angles of: 3.3±0.2°, 7.6±0.2°, and 8.5±0.2°.

**15.** The crystal form C according to claim 14, wherein the X-ray powder diffraction pattern has characteristic diffraction peaks at 2θ angles of 3.3±0.2°, 7.6±0.2°, 8.5±0.2°, 11.3±0.2°, 13.8±0.2°, 15.2±0.2°, 17.7±0.2°, and 18.3±0.2°.

**16.** The crystal form C according to claim 15, wherein the X-ray powder diffraction pattern has characteristic diffraction peaks at 2θ angles of 3.3±0.2°, 7.6±0.2°, 8.5±0.2°, 11.3±0.2°, 13.8±0.2°, 15.2±0.2°, 17.7±0.2°, 18.3±0.2°, 22.6±0.2°, 23.7±0.2°, 24.9±0.2°, and 26.6±0.2°.

**17.** The crystal form C according to claim 16, wherein the XRPD pattern is as shown in FIG. 4.

**18.** A crystal form D of the compound of formula (III), which is **characterized in that** it has an X-ray powder diffraction pattern having characteristic diffraction peaks at 2θ angles of: 3.5±0.2°, 9.5±0.2°, and 10.5±0.2°.

**19.** The crystal form D according to claim 18, wherein the X-ray powder diffraction pattern has characteristic diffraction peaks at 2θ angles of 3.5±0.2°, 9.5±0.2°, 10.5±0.2°, 14.0±0.2°, 16.3±0.2°, 18.7±0.2°, 23.4±0.2°, and 25.6±0.2°.

**20.** The crystal form D according to claim 19, wherein the XRPD pattern is as shown in FIG. 5.

**21.** A compound of formula (IV),

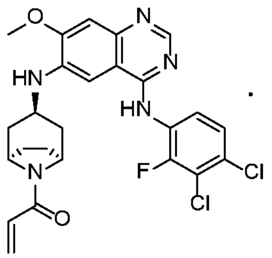

· $H_2SO_4$

(IV)

.

**22.** A crystal form E of the compound of formula (IV), which is **characterized in that** it has an X-ray powder diffraction pattern having characteristic diffraction peaks at 2θ angles of: 3.3±0.2°, 12.8±0.2°, and 13.2±0.2°.

**23.** The crystal form E according to claim 22, wherein the X-ray powder diffraction pattern has characteristic diffraction peaks at 2θ angles of 3.3±0.2°, 8.1±0.2°, 13.2±0.2°, 14.9±0.2°, 16.2±0.2°, 18.7±0.2°, 23.2±0.2°, and 24.8±0.2°.

**24.** The crystal form E according to claim 23, wherein the XRPD pattern is as shown in FIG. 6.

**25.** A crystal form F of the compound of formula (IV), which is **characterized in that** it has an X-ray powder diffraction pattern having characteristic diffraction peaks at 2θ angles of: 3.6±0.2°, 7.5±0.2°, and 16.6±0.2°.

**26.** The crystal form F according to claim 25, wherein the X-ray powder diffraction pattern has characteristic diffraction peaks at 2θ angles of 3.6±0.2°, 7.5±0.2°, 12.2±0.2°, 15.1±0.2°, 16.6±0.2°, 23.6±0.2°, and 24.6±0.2°.

**27.** The crystal form F according to claim 26, wherein the XRPD pattern is as shown in FIG. 7.

**28.** A method for preparing the crystal form A of the compound of formula (I), comprising:

(1) adding a compound of formula (I) in a solvent to dissolve the compound of formula (I); and
(2) heating with stirring, cooling, filtering, and drying to give the crystal form A of the compound of formula (I).

**29.** A method for preparing the crystal form B of the compound of formula (II), comprising:

(1) adding a compound of formula (I) in a solvent to dissolve the compound of formula (I); and
(2) heating, adding maleic acid, stirring, precipitating solid, cooling, filtering, and drying to give the crystal form B of the compound of formula (II).

**30.** The preparation method according to claim 28, wherein the solvent is tetrahydrofuran.

**31.** The preparation method according to claim 29, wherein the solvent is selected from ethanol and ethyl acetate.

**32.** The preparation method according to claim 28 or 29, wherein the weight-volume ratio of the compound to the solvent ranges from 1g : 7 to 50mL.

**33.** Use of the compound according to any one of claims 6, 13 or 21 or the crystal form according to any one of claims 1 to 5, 7 to 12, 14 to 20, or 22 to 27 or the crystal form obtained by the preparation method according to any one of claims 28 to 32 in the manufacture of a medicament for treating a disease related to Pan-HER tyrosine kinase inhibitor.

**34.** The use according to claim 33, which is **characterized in that** the medicament related to Pan-HER tyrosine kinase inhibitor is used for a tumor.

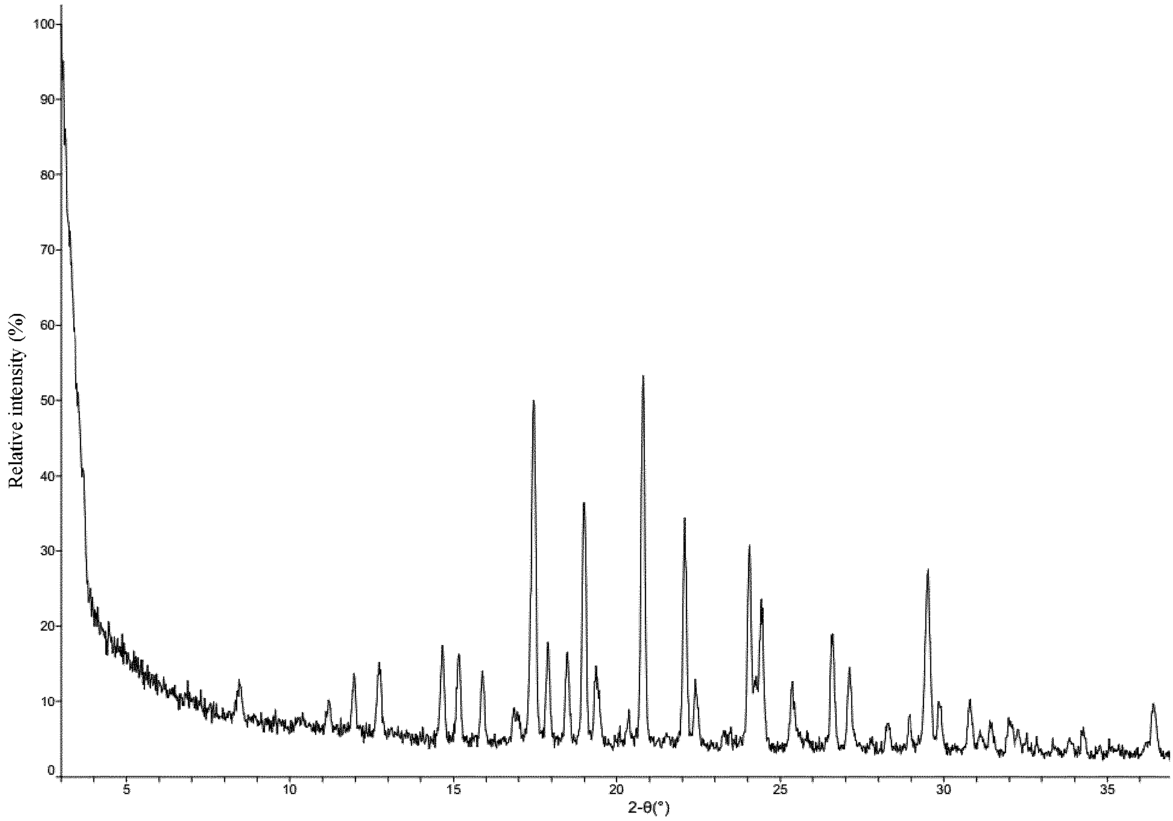
Relative intensity (%)
2-θ(°)
0
10
20
30
40
50
60
70
80
90
100
5
10
15
20
25
30
35

FIG. 1

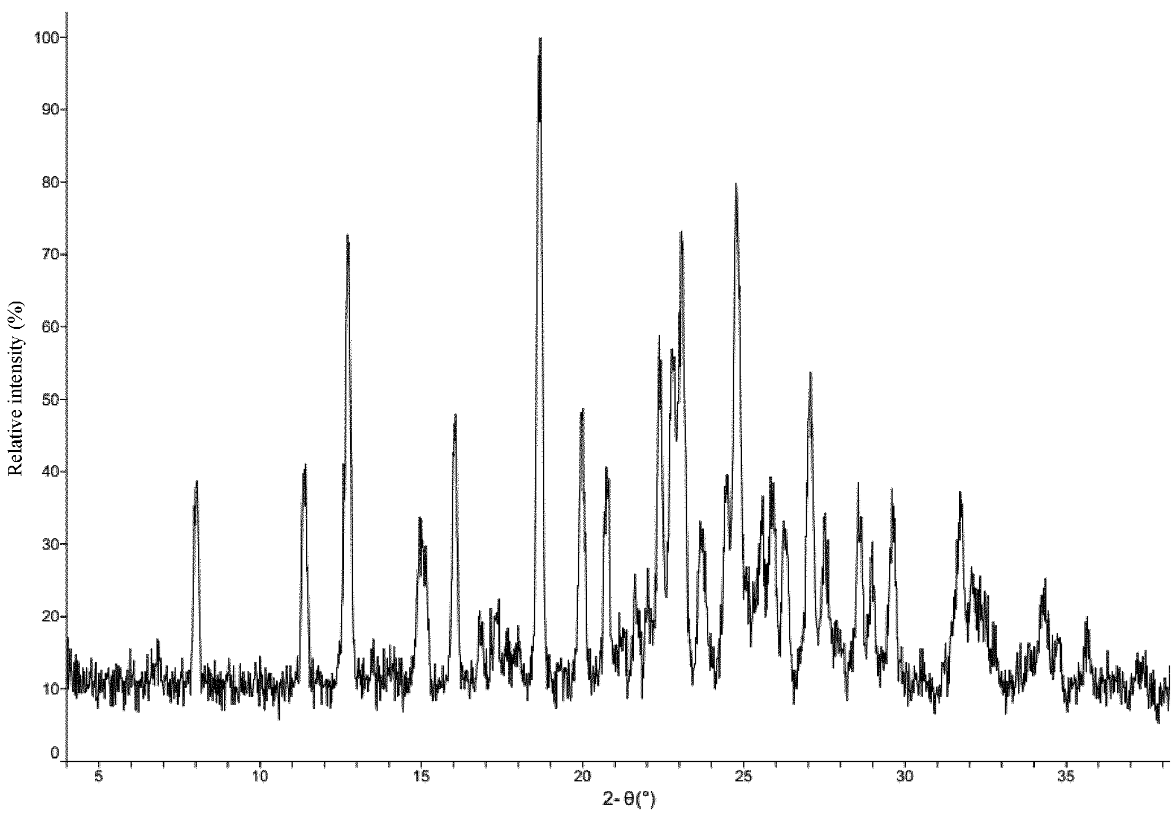
Relative intensity (%)
2- θ(°)
0
10
20
30
40
50
60
70
80
90
100
5
10
15
20
25
30
35

FIG. 2

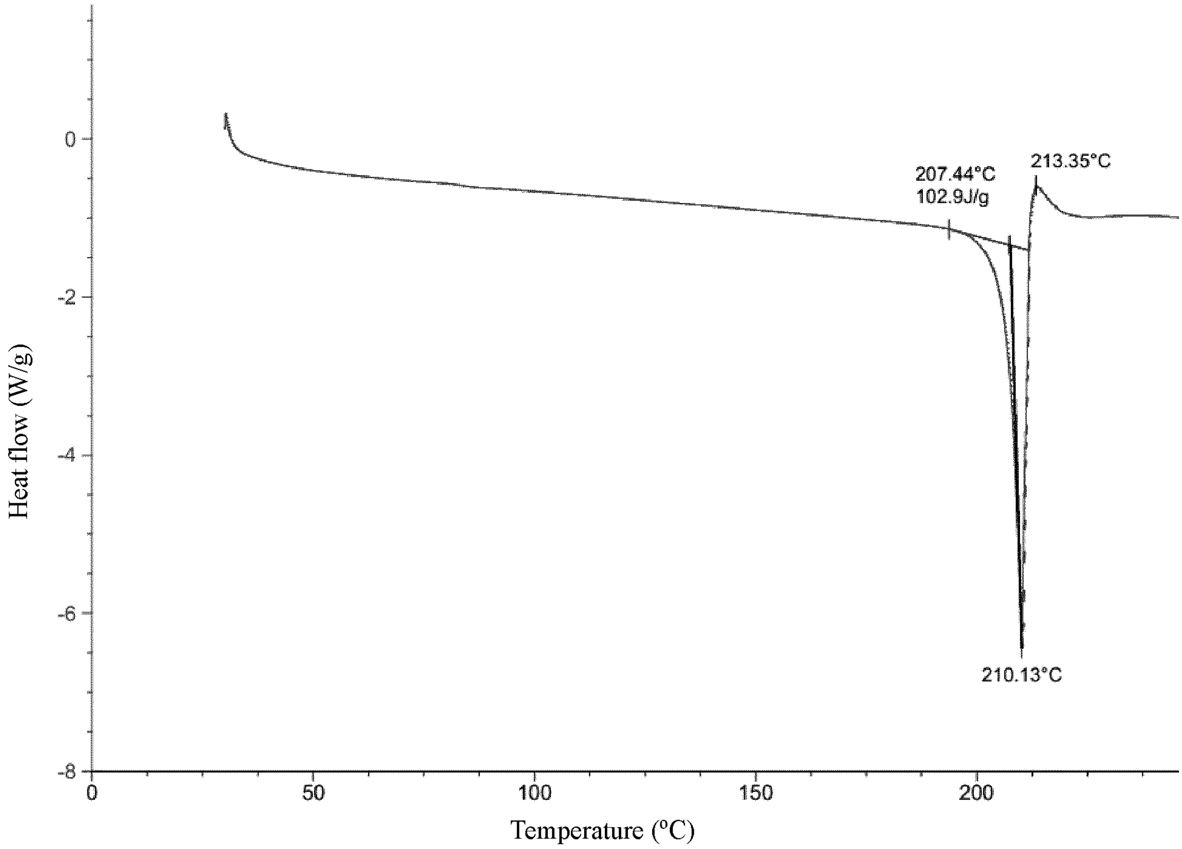
Heat flow (W/g)
0
-2
-4
-6
-8
207.44°C
102.9J/g
213.35°C
210.13°C
0
50
100
150
200
Temperature (°C)

FIG. 3

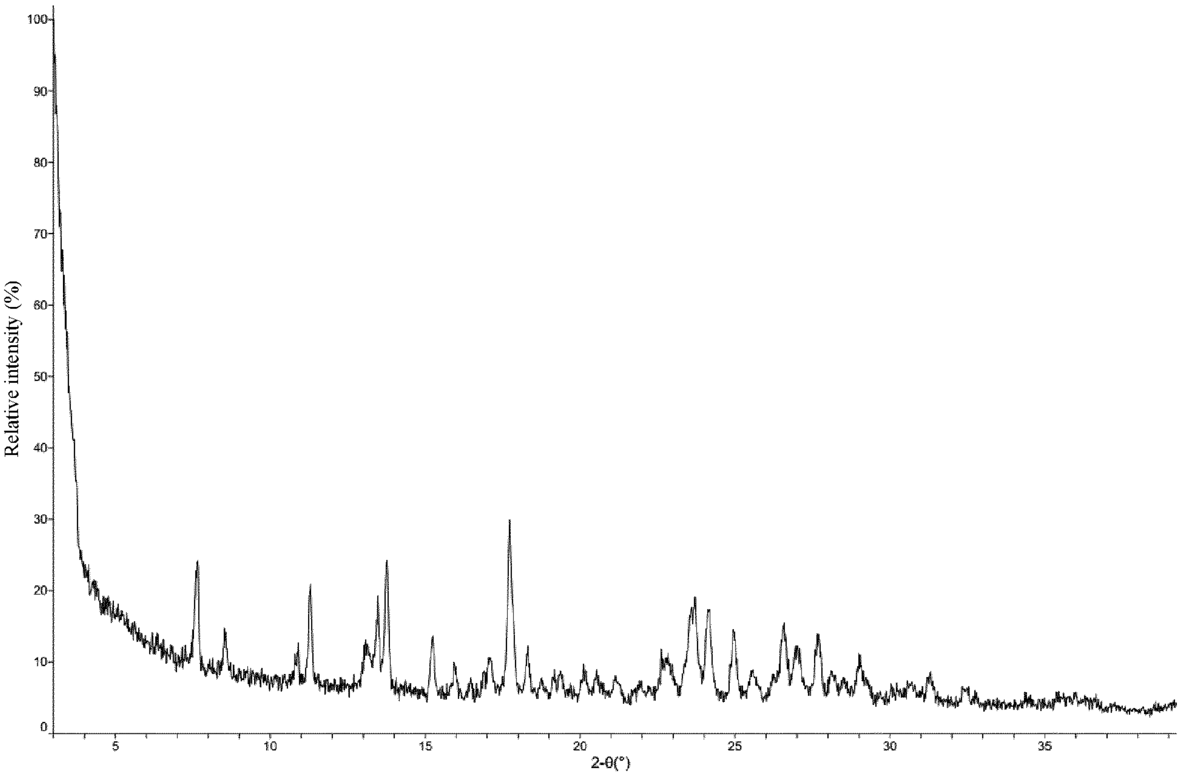
Relative intensity (%)
100
90
80
70
60
50
40
30
20
10
0
5
10
15
20
25
30
35
2-θ(°)

FIG. 4

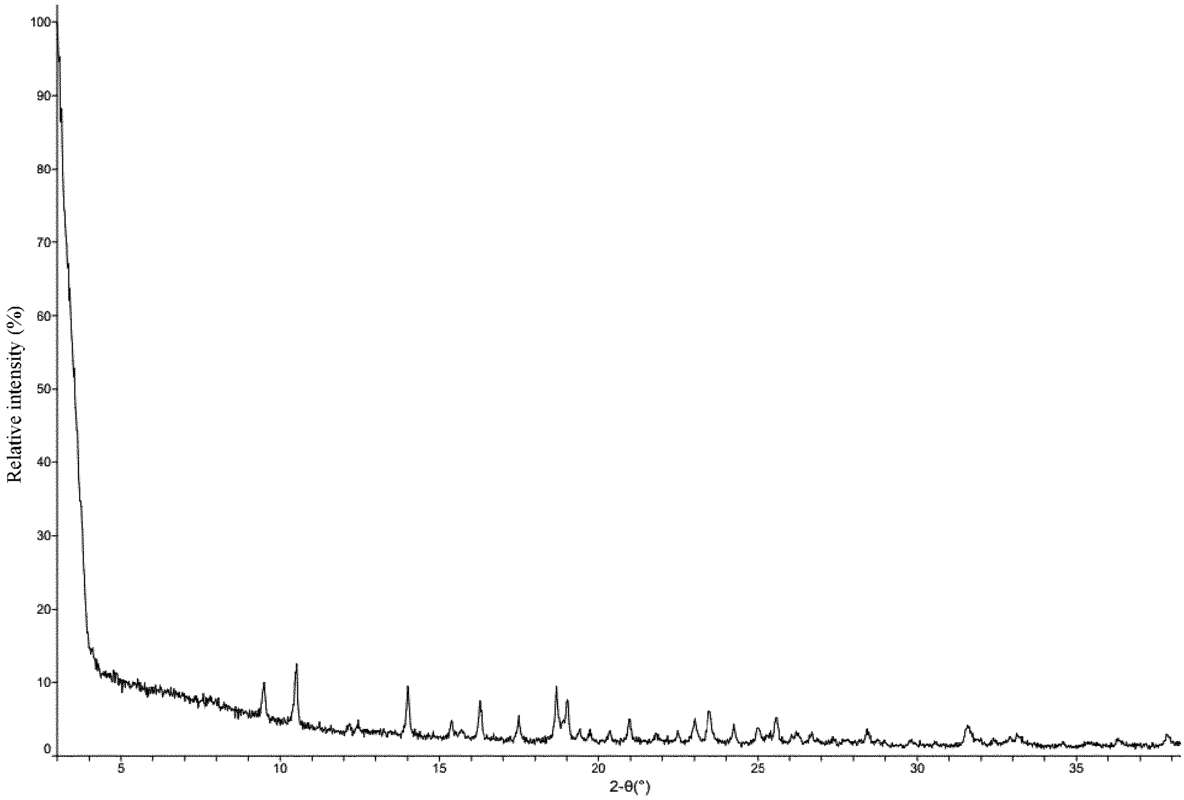
Relative intensity (%)
2-θ(°)
100
90
80
70
60
50
40
30
20
10
0
5
10
15
20
25
30
35

FIG. 5

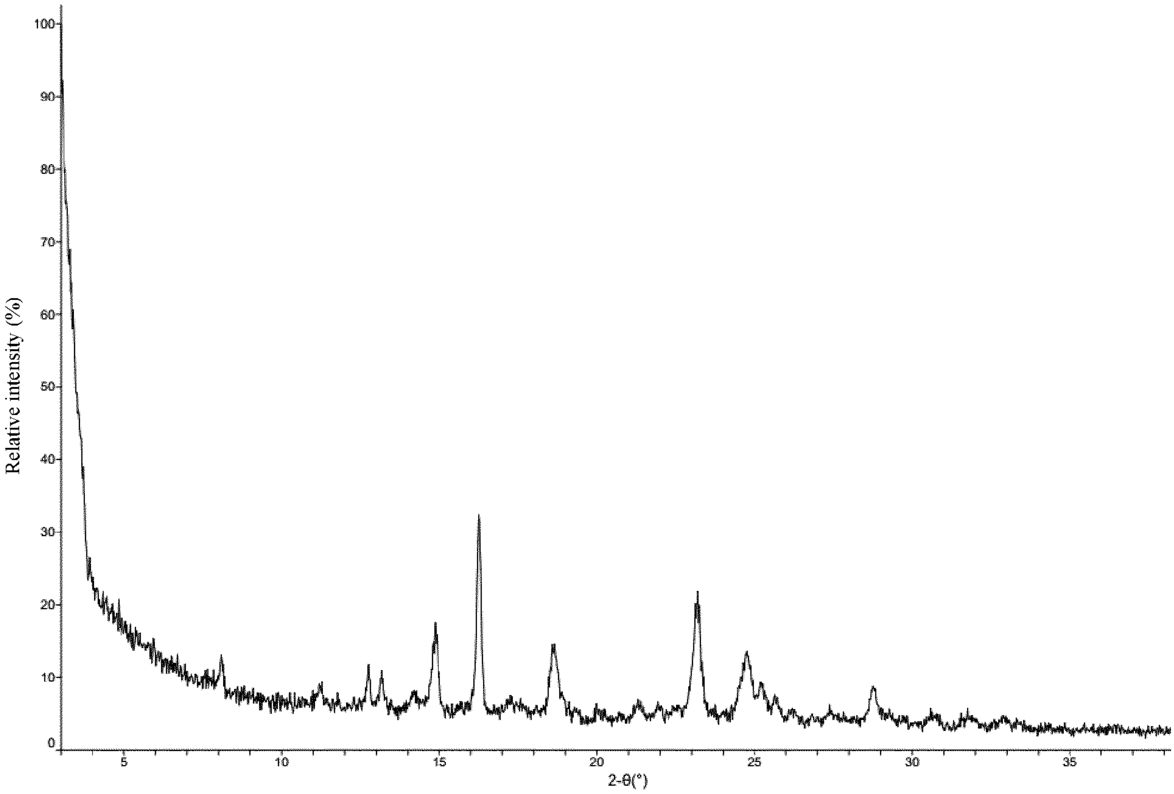
Relative intensity (%)
2-θ(°)
100
90
80
70
60
50
40
30
20
10
0
5
10
15
20
25
30
35

FIG. 6

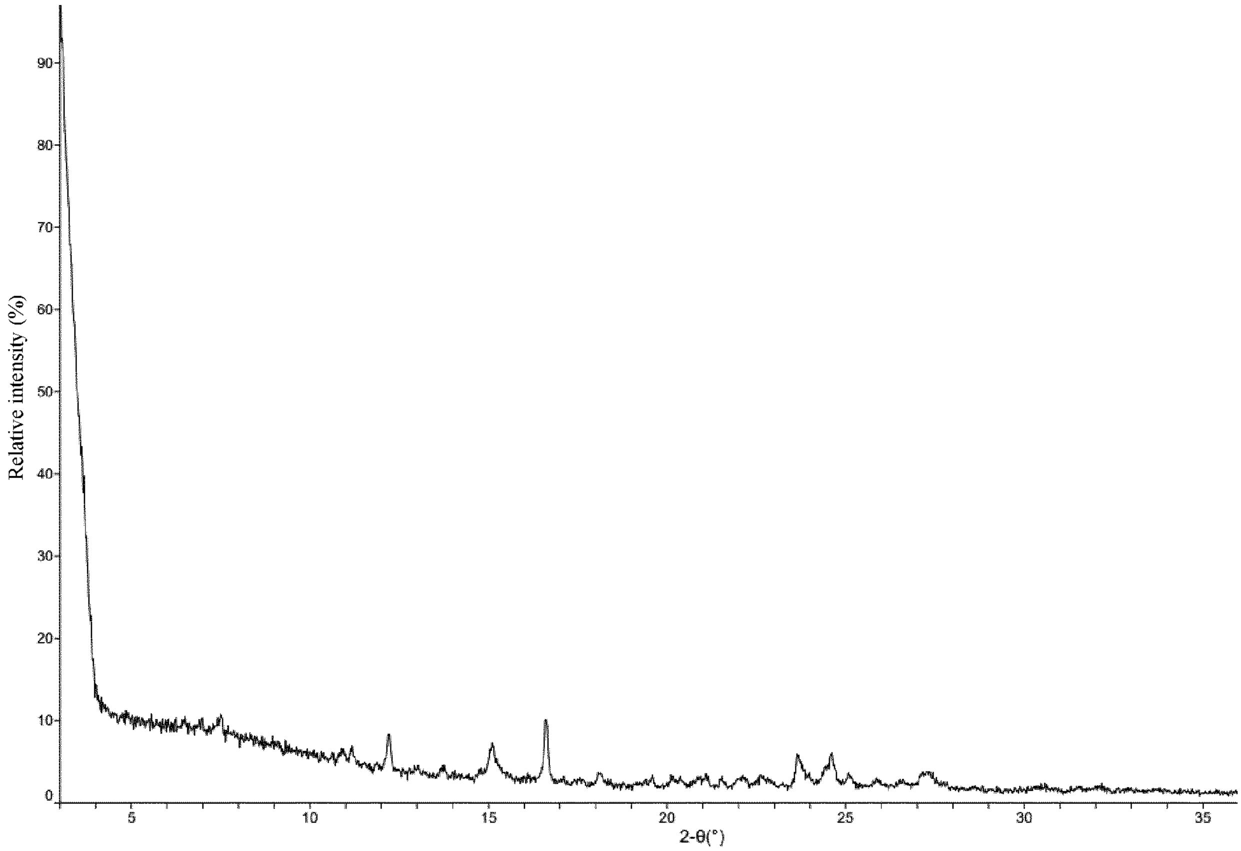
Relative intensity (%)
90
80
70
60
50
40
30
20
10
0
5
10
15
20
25
30
35
2-θ(°)

FIG. 7

**INTERNATIONAL SEARCH REPORT**

International application No.
**PCT/CN2020/097158**

**A. CLASSIFICATION OF SUBJECT MATTER**

C07D 498/04(2006.01)i; A61K 31/517(2006.01)i; A61P 35/00(2006.01)i; C07D 403/12(2006.01)i; C07D 405/14(2006.01)i

According to International Patent Classification (IPC) or to both national classification and IPC

**B. FIELDS SEARCHED**

Minimum documentation searched (classification system followed by classification symbols)

C07D 498/-; C07D 403/-; C07D 405/-; A61K 31/-; A61P 35/-

Documentation searched other than minimum documentation to the extent that such documents are included in the fields searched

Electronic data base consulted during the international search (name of data base and, where practicable, search terms used)

CNABS, CNKI, DWPI, SIPOABS, STN(REG,CAPLUS)结构式检索, STN(REG,CAPLUS) STRUCTURAL FORMULA SEARCH, GOOGLE Scholar, Pubmed, CNTXT, USTXT, EPTXT, 中国药物专利数据库,CTCMPD: 南京明德, 药明康德, 韩美, 正大天晴, 陈新海, 张丽, 周凯, 于衍新, 胡伯羽, 章晖宇, 喹唑啉, 晶体, 晶型, 表皮生长因子, 酪氨酸激酶, 抑制剂, 波齐替尼, 增生, 肿瘤, 癌, quinazolinyl, quinazolyl, quinazolin???, crystal?, EGF, EGFR, epidermal, growth, factor, inhibit+, kinase, tyrosin+, tyrosine kinase inhibitor, Pan-HER, HER-2, HER-2/neu, tumor,cancer, Poziotinib, HM781-36b, NOV120101, CAS RN: 2354388-66-0, 1092364-38-9

**C. DOCUMENTS CONSIDERED TO BE RELEVANT**

| Category* | Citation of document, with indication, where appropriate, of the relevant passages | Relevant to claim No. |
|---|---|---|
| PX | WO 2019120213 A1 (MEDSHINE DISCOVERY INC.) 27 June 2019 (2019-06-27)<br>claims 1 and 24-26, description, pages 1 and 2, page 23, paragraph 5, and embodiment 38, evaluation experiment in vitro | 1-34 |
| A | CN 101679384 A (HANMI PHARMACEUTICAL CO., LTD.) 24 March 2010 (2010-03-24)<br>description, page 3, paragraph 6 to page 4, paragraph 1 , embodiment 36, page 72, table number 36, and page 16, paragraph 2 | 1-34 |
| A | CN 105555782 A (CHIA TAI TIANQING PHARMACEUTICAL GROUP CO., LTD. et al.) 04 May 2016 (2016-05-04)<br>description, paragraphs [0007]-[0019], embodiment 2, and paragraph [0045] | 1-34 |
| A | CN 105461729 A (RUYUAN YAO AUTONOMOUS COUNTY DAZHONG MEDICINE TRADE CO., LTD.) 06 April 2016 (2016-04-06)<br>claim 1 | 1-34 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **05 August 2020** | **24 September 2020** |
| Name and mailing address of the ISA/CN<br>**China National Intellectual Property Administration (ISA/CN)**<br>**No. 6, Xitucheng Road, Jimenqiao Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/097158**

**C. DOCUMENTS CONSIDERED TO BE RELEVANT**

| Category* | Citation of document, with indication, where appropriate, of the relevant passages | Relevant to claim No. |
|---|---|---|
| A | CN 105294717 A (RUYUAN YAO AUTONOMOUS COUNTY DAZHONG MEDICINE TRADE CO., LTD.) 03 February 2016 (2016-02-03)<br>claim 1 | 1-34 |
| A | CN 106588943 A (GUANGDONG HEC PHARMACEUTICAL CO., LTD.) 26 April 2017 (2017-04-26)<br>claim 1 | 1-34 |
| A | ROSKOSKI Robert Jr. "ErbB/HER Protein-tyrosine kinases: Structures and Small Molecule Inhibitors"<br>*Pharmacological Research,* Vol. 87, 30 September 2014 (2014-09-30),<br>pp. 42-59 | 1-34 |

Form PCT/ISA/210 (second sheet) (January 2015)

**INTERNATIONAL SEARCH REPORT**
**Information on patent family members**

International application No.
**PCT/CN2020/097158**

| Patent document cited in search report | Publication date (day/month/year) | Patent family member(s) | Publication date (day/month/year) |
|---|---|---|---|
| WO 2019120213 A1 | 27 June 2019 | None | |
| CN 101679384 A | 24 March 2010 | RU 2434010 C2 | 20 November 2011 |
| | | US 8188102 B2 | 29 May 2012 |
| | | HK 1142591 A1 | 16 May 2014 |
| | | AU 2008260772 A1 | 11 December 2008 |
| | | JP 5155391 B2 | 06 March 2013 |
| | | UA 96045 C2 | 26 September 2011 |
| | | DK 2167492 T3 | 11 January 2016 |
| | | JP 2010529115 A | 26 August 2010 |
| | | HU E028243 T2 | 28 December 2016 |
| | | TW I377944 B | 01 December 2012 |
| | | TW 200906414 A | 16 February 2009 |
| | | MY 144972 A | 30 November 2011 |
| | | EP 2167492 B1 | 14 October 2015 |
| | | BR PI0811069 A2 | 09 December 2014 |
| | | AR 110003 A2 | 13 February 2019 |
| | | HR P20160007 T1 | 12 February 2016 |
| | | RU 2009149307 A | 20 July 2011 |
| | | CA 2687180 C | 24 July 2012 |
| | | WO 2008150118 A2 | 11 December 2008 |
| | | NZ 582412 A | 22 December 2011 |
| | | AU 2008260772 B2 | 02 June 2011 |
| | | CN 101679384 B | 16 October 2013 |
| | | PL 2167492 T3 | 31 March 2016 |
| | | WO 2008150118 A3 | 29 January 2009 |
| | | AR 066876 A1 | 16 September 2009 |
| | | PT 2167492 E | 04 February 2016 |
| | | KR 20080107294 A | 10 December 2008 |
| | | IL 202154 A | 28 May 2014 |
| | | MX 2009012772 A | 15 December 2009 |
| | | EP 2167492 A4 | 15 December 2010 |
| | | IL 202154 D0 | 16 June 2010 |
| | | CA 2687180 A1 | 11 December 2008 |
| | | EP 2167492 A2 | 31 March 2010 |
| | | US 2010179120 A1 | 15 July 2010 |
| | | KR 101013319 B1 | 09 February 2011 |
| | | ES 2558623 T3 | 05 February 2016 |
| | | ZA 201000022 B | 28 April 2011 |
| CN 105555782 A | 04 May 2016 | EA 034573 B1 | 21 February 2020 |
| | | KR 20160058946 A | 25 May 2016 |
| | | KR 101843752 B1 | 30 March 2018 |
| | | AU 2014327932 B2 | 03 November 2016 |
| | | HK 1221720 A1 | 09 June 2017 |
| | | EP 3050880 B1 | 19 June 2019 |
| | | BR 112016006692 A2 | 12 May 2020 |
| | | AU 2014327932 A1 | 21 April 2016 |
| | | CN 104513229 A | 15 April 2015 |
| | | CN 105555782 B | 10 November 2017 |
| | | JP 2016531937 A | 13 October 2016 |
| | | JP 6207752 B2 | 04 October 2017 |

Form PCT/ISA/210 (patent family annex) (January 2015)

**INTERNATIONAL SEARCH REPORT**
**Information on patent family members**

International application No.
**PCT/CN2020/097158**

| Patent document cited in search report | Publication date (day/month/year) | Patent family member(s) | Publication date (day/month/year) |
|---|---|---|---|
| | | CN 107556295 A | 09 January 2018 |
| | | EP 3050880 A4 | 26 April 2017 |
| | | AU 2014327932 B9 | 13 April 2017 |
| | | WO 2015043515 A1 | 02 April 2015 |
| | | EA 201600204 A1 | 31 October 2016 |
| | | US 9725439 B2 | 08 August 2017 |
| | | US 2016214964 A1 | 28 July 2016 |
| | | CA 2924705 C | 17 October 2017 |
| | | CA 2924705 A1 | 02 April 2015 |
| | | EP 3050880 A1 | 03 August 2016 |
| CN 105461729 A | 06 April 2016 | CN 105461729 B | 05 December 2017 |
| CN 105294717 A | 03 February 2016 | CN 105294717 B | 22 December 2017 |
| CN 106588943 A | 26 April 2017 | EP 3365344 A1 | 29 August 2018 |
| | | KR 20180064533 A | 14 June 2018 |
| | | CA 3001655 A1 | 27 April 2017 |
| | | EP 3365344 A4 | 22 May 2019 |
| | | AU 2016343517 A1 | 24 May 2018 |
| | | US 2018298019 A1 | 18 October 2018 |
| | | WO 2017067447 A1 | 27 April 2017 |
| | | CN 106588943 B | 16 October 2018 |
| | | US 10308658 B2 | 04 June 2019 |
| | | AU 2016343517 B2 | 30 April 2020 |
| | | TW 201718596 A | 01 June 2017 |
| | | JP 2018534289 A | 22 November 2018 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 201910533240
- WO 2008150118 A
- WO 2015043515 A


### Non-patent literature cited in the description


- **BASELGA. J.** *Oncologist,* 2002, vol. 7, 2-8